# EUROPEAN PATENT APPLICATION

(11) **EP 4 831 277 A1**
(43) Date of publication of application: **28.10.2026**
(21) Application number: 24907520.1
(22) Date of filing: 19.12.2024
(51) Int. Cl.: C12N 5/071, A61K 35/36, A61P 17/00, A61P 43/00, C12N 5/10, C12Q 1/6897

(54) **METHOD FOR PRODUCING NAIL MATRIX CELLS**

(30) Priority: 20.12.2023 JP 2023214532
(71) Applicant: Kansai Medical University Educational Corporation, City of Hirakata, Osaka 573-1010 (JP)
(72) Inventor: HATTORI, Fumiyuki, Hirakata City, Osaka 5731010 (JP)
(74) Representative: J A Kemp LLP
(86) International application number: PCT/JP2024/045067
(87) International publication number: WO 2025/135136

(57) **Abstract**

The present invention provides a method for producing nail matrix cells, the method comprising a step of culturing a nail stem cell by shaking under suspension culture conditions in the presence of a Wnt signaling activator, an FGF signaling activator, and an adrenocortical hormone.

## Description

### [TECHNICAL FIELD]

The present invention relates to a method for producing nail matrix cells, the nail matrix cells produced by the method, and an agent for treating fingertip disorders, comprising the cells. The present invention also relates to nail matrix cells comprising a reporter gene, and a method for screening prophylactic or therapeutic medicament for fingertip disorders using the cells.

### [BACKGROUND ART]

Nails are evolutionarily conserved structures found in all known terrestrial mammals. In particular, human nails are important for finger function, sensation, protection, and aesthetics. Congenital malformations, fingertip injuries, and anticancer chemotherapy can cause intractable nail abnormalities, but there is no effective treatment for severe cases.

The nail matrix, which is a stem cell or progenitor cell population of the nail, produces nails throughout life. The nail matrix divides rapidly in an asymmetric manner, and its daughter cells gradually differentiate and keratinize to form mature nails. Embryologically, the finger primordia (initially called the limb bud) develops from the outer body wall on day 24 of gestation. The limb bud has a mesenchymal core and an epithelial cap, and is elongated to form arms and legs by stimulation with FGF8 and Wnt proteins secreted by the apical ectodermal ridge (AER), which is similar to the amphibian blastocyst, and then segmented. As segmentation proceeds, segment-specific expression of homeobox genes occurs, and the most distal last segment expressing Hoxd13 forms the fingers. The nail primordium appears as a thickening of the epidermis near the dorsal distal end of the finger around 10 weeks of gestation. The nail matrix is formed from the proximal portion of the nail fold at about 16 weeks.

Previously, it has been reported that limb buds capable of differentiating into forelimbs and hind limbs were successfully generated from mouse pluripotent stem cells (Non-Patent Literature 1). However, to date, there have been no reports on the production of nail matrix cells from pluripotent stem cells.

### [CITATION LIST]

### [NON PATENT LITERATURE]

[NPL 1] Mori S, et al., Nat Commun. 2019;10: 3802

### [SUMMARY OF INVENTION]

### [TECHNICAL PROBLEM]

Therefore, an object of the present invention is to provide a method for producing nail matrix cells. Another object of the present invention is to provide the nail matrix cells produced by the method, an agent for treating fingertip disorders such as nail disorders, comprising the cells, and a method for screening prophylactic or therapeutic substances for fingertip disorders such as nail disorders using the cells.

### [SOLUTION TO PROBLEM]

The present inventor conceived that further differentiation of limb bud organoids described in the above-cited Non-Patent Literature 1 could yield organoids containing nail matrix cells. Therefore, the present inventor attempted to induce differentiation from pluripotent stem cells into limb bud organoids using the method described in Non-Patent Literature 1, but unexpectedly found a large number of cell deaths. To prevent cell death, the present inventor modified the method by culturing the cells for a certain period during early differentiation and then gradually changing the environment to allow the cells to adapt, which successfully induced limb bud organoids efficiently. Furthermore, after repeated trials to further differentiate the thus-induced limb bud organoids, the present inventor succeeded in differentiating the organoid into digit organoids by culturing it under suspension conditions with shaking in the presence of Wnt signaling activators, FGF signaling activators, and adrenocortical hormones. The present inventor concluded that the digit organoids produced in this way contain nail matrix cells and primitive nails. To the best of the inventors' knowledge, there have been no reports on the induction of nail matrix cells *in vitro*, and this success was surprising.

Furthermore, in the digit organoid, the present inventor found that Lgr5 protein-expressing cells exist near Lgr6 protein-expressing cells, and some cells co-express both proteins. From these results, the present inventor concluded that there is a strong relationship between Lgr5-positive cells and Lgr6-positive cells, and it is highly likely that the Lgr6-positive nail matrix cells contained in the digit organoid are derived from Lgr5-positive nail stem cells. Furthermore, the present inventor designed a reporter gene under the control of the transcriptional regulatory region of the Lgr6 gene, and differentiated pluripotent stem cells into digit organoids containing nail matrix cells, and confirmed that the reporter protein was also expressed in cells expressing Lgr6. Based on these findings, the present inventor conducted further research and completed the present invention.

That is, the present invention is as follows:
[1] A method for producing a nail matrix cell, comprising a step of culturing a nail stem cell by shaking under suspension culture conditions in the presence of a Wnt signaling activator, an FGF signaling activator, and an adrenocortical hormone.
[2] The method according to [1], wherein the nail stem cell is contained in a limb bud organoid.
[3] The method according to [1] or [2], wherein the nail matrix cell is contained in a digit organoid.
[4] The method according to any one of [1] to [3], wherein the Wnt signaling activator is CHIR98014.
[5] The method according to any one of [1] to [4], wherein the FGF signaling activator is bFGF and/or FGF8.
[6] The method according to any one of [1] to [5], wherein the adrenocortical hormone is hydrocortisone.
[7] The method according to any one of [1] to [6], wherein the nail stem cell is derived from a pluripotent stem cell.
[8-1] The method according to [7], wherein the pluripotent stem cell is derived from a human.
[8-2] The method according to any one of [1] to [8-1], wherein the nail stem cell is derived from a human.
[9] A nail matrix cell or a digit organoid, comprising a cell producible by the method according to any one of [1] to [8-2].
[10] A therapeutic agent for fingertip disorders, comprising the nail matrix cell or the digit organoid according to [9].
[11] The therapeutic agent according to [10], wherein the fingertip disorders are nail disorders.
[12] A nail matrix cell having a nucleic acid comprising a reporter gene under the control of a transcriptional regulatory region of an Lgr6 gene.
[13] The nail matrix cell according to [12], producible by the method according to any one of [1] to [8-2].
[14] The nail matrix cell according to [12] or [13], wherein the reporter gene is a gene encoding a fluorescent protein.
[15] A method for screening a prophylactic or therapeutic medicament for fingertip disorders, comprising the following steps (1) to (3):
   (1) contacting the nail matrix cell according to any one of [12] to [14] with a test substance,
   (2) measuring the expression level of the reporter protein in the cell, and
   (3) selecting the test substance as a candidate for a prophylactic or therapeutic medicament for fingertip disorders when the expression level measured in step (2) is higher than that in a control group not contacted with the test substance, or higher than that measured in the absence of the test substance.
[16] The method according to [15], wherein the fingertip disorders are nail disorders.
[17] A method for treating fingertip disorders, comprising transplanting an effective amount of the nail matrix cells or the digit organoid according to [9] into a subject.
[18] The nail matrix cell or the digit organoid according to [9] for use in the treatment of fingertip disorders.
[19] Use of the nail matrix cell or the digit organoid according to [9] in the manufacture of a therapeutic medicament for fingertip disorders.

### [ADVANTAGEOUS EFFECTS OF INVENTION]

The present invention makes it possible to provide nail matrix cells or digit organoids *in vitro*, and enables the development of regenerative medicine by transplanting the nail matrix cells or digit organoid into patients with fingertip disorders such as nail disorders. In particular, nail regeneration can be very important for quality of life (QOL) in patients with nail disorders. It is also possible to provide a digit organoid with a low risk of tumorigenesis due to contamination with undifferentiated cells during transplantation into a living body. Furthermore, the present invention provides nail matrix cells containing a nucleic acid comprising a reporter gene under the control of the transcriptional regulatory region of the Lgr6 gene, and makes it possible to screen for prophylactic or therapeutic medicaments for fingertip disorders using the nail matrix cell.

### [BRIEF DESCRIPTION OF DRAWINGS]

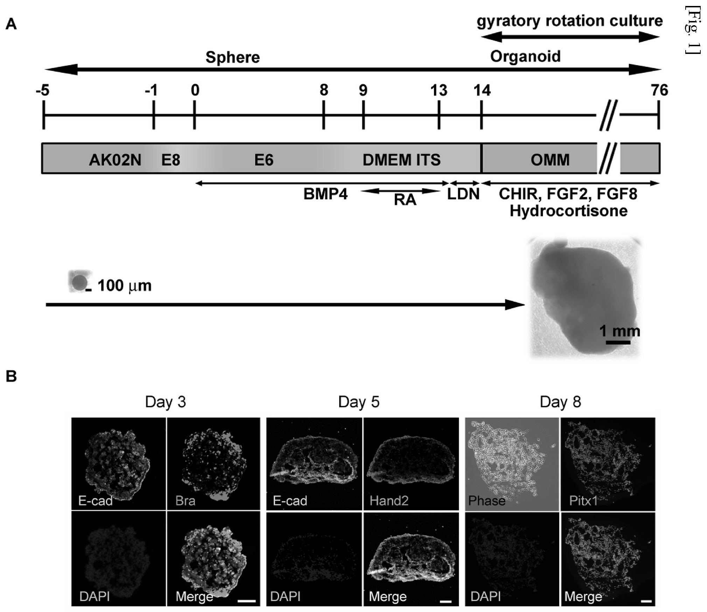

[FIG. 1] FIG. 1 shows the differentiation scheme of the digit organoid and the early expression of limb bud-related proteins. (A) Differentiation scheme. (B) Immunofluorescent staining of early spheres. Scale bar = 200 µm.

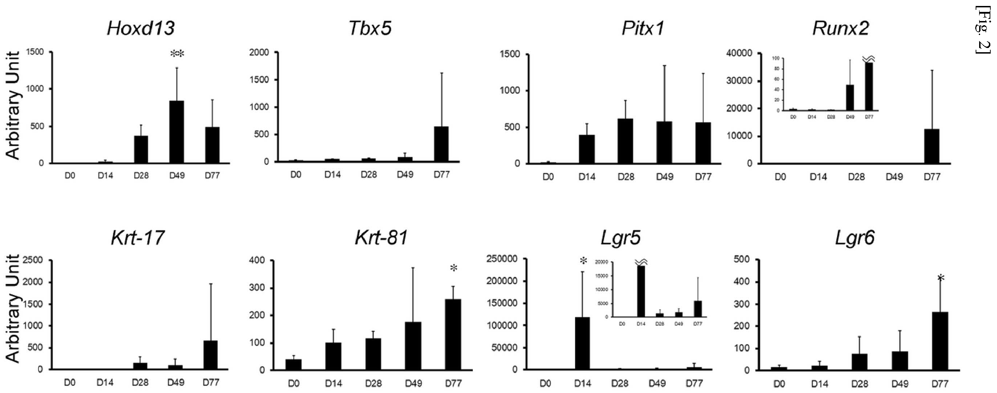

[FIG. 2] FIG. 2 shows time course of the gene expressions from pluripotent to digit organoid via limb bud formation. The gene expression levels were normalized to internal ribosomal protein S18 expression levels. **p < 0.01, *p < 0.05. Data are shown as mean ± SD.

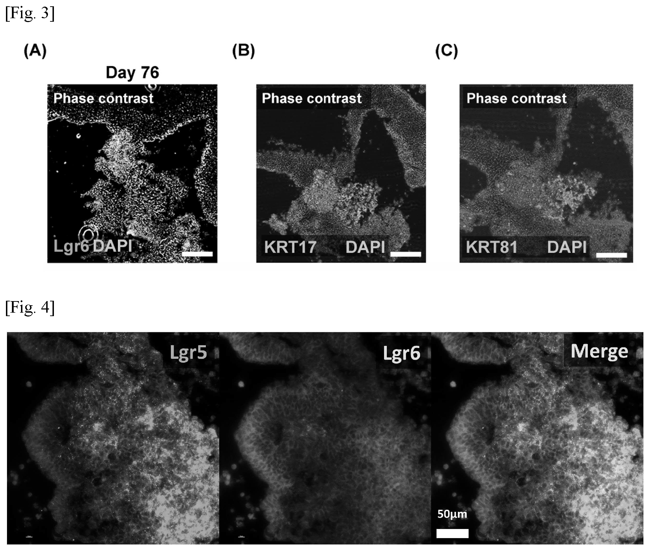

[FIG. 3] FIG. 3 shows immunohistochemical detection of Lgr6 and hard keratins. Immunohistochemical staining detected the expression of (A) Lgr6, (B) Keratin 17, and (C) Keratin 81. Scale bar = 200 µm.
[FIG. 4] FIG. 4 shows immunohistochemical detection of Lgr5 and Lgr6. Immunohistochemical staining detected that Lgr5 protein-expressing cells exist in the vicinity of Lgr6 protein-expressing cells, and that cells co-expressing both also exist. Scale bar = 50 µm.

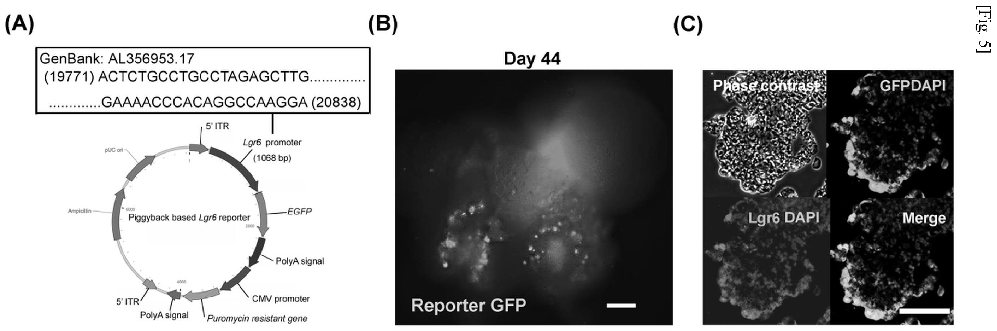

[FIG. 5] FIG. 5 shows functionality of the Lgr6-GFP reporter iPSC. (A) Design of the Lgr6-GFP reporter gene on the piggyBac system (B) Expression of reporter GFP on day 44 of organoid culture. (C) Immunohistochemical detection of Lgr6 protein and reporter GFP. Scale bars = 200 µm. The sequences shown from top to bottom are listed in the sequence listing as SEQ ID NOs. 20 and 21.

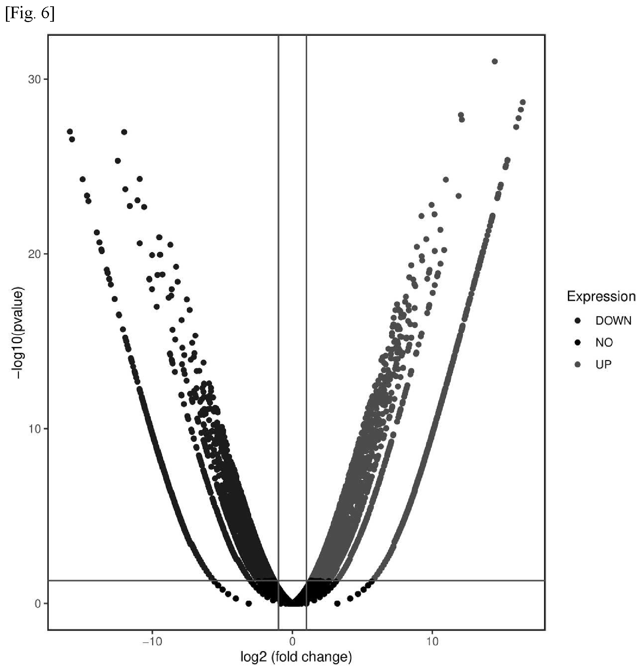

[FIG. 6] FIG. 6 is an overview of a comprehensive comparative gene expression analysis between undifferentiated iPS cells and digit organoids. RNA sequencing was used to quantify mRNA expression levels for each gene, and fold change (x-axis) and significance (P value) were compared. A negative log2(fold change) indicates higher expression in iPS cells, whereas a positive value indicates higher expression in digit organoids. In addition, the higher the vertical axis (the larger the -log10 value), the smaller the P value and the greater the statistical significance.

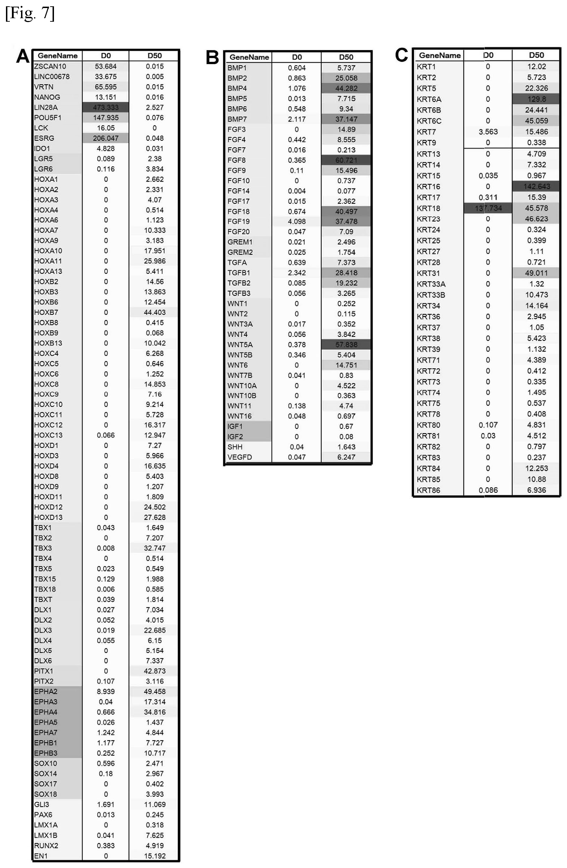

[FIG. 7] FIG. 7 shows FPKM (fragments per kilobase of exon per million mapped reads) values. The relative abundance of each gene, normalized by RNA sequencing data, is shown in a table. Transcriptional regulation-related genes involved in limb and digit development are shown in A, extracellular growth factor-related genes in B, and keratin family genes in C.

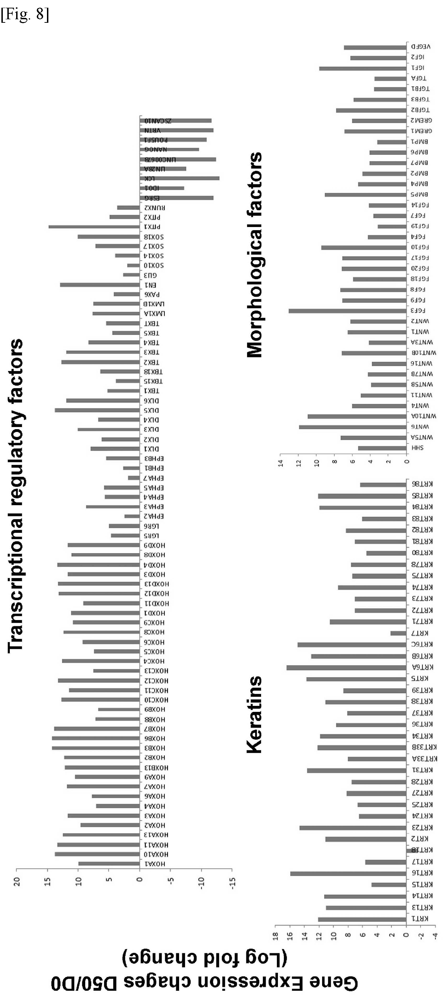

[FIG. 8] FIG. 8 shows the results of a comparative analysis of specific gene expression levels between undifferentiated iPS cells and digit organoids by RNA sequencing. For the genes shown in Fig. 7, expression levels in digit organoids were compared with those in iPS cells, and the results are shown as a bar graph with log2 (fold change) on the y-axis.

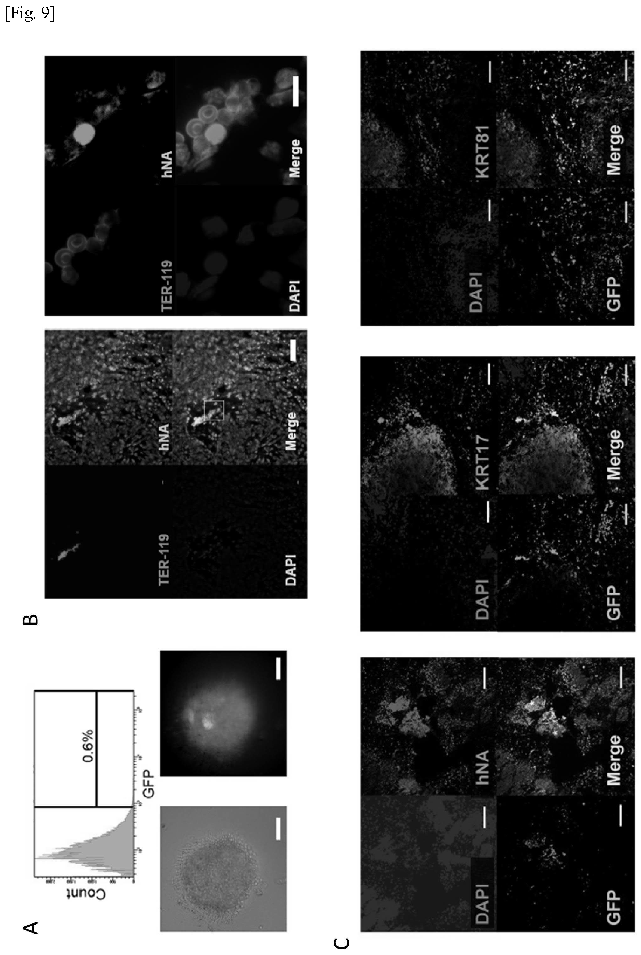

[FIG. 9] Purification of Lgr6-expressing cells, preparation of cell aggregates, and transplantation into subcutaneous tissue of the back and engraftment. A shows a figure in which Lgr6 reporter iPS cells prepared in Example 5 were differentiated into digit organoids by the methods described in Examples 1 and 2, and cells showing fluorescence (GFP-positive cells) were collected using a cell sorter (FACS Aria IIIBD), and the cell aggregate prepared in a nonadherent 96-well round-bottom flask (SUMILON MS-9096U, M) is shown (scale bar = 100 µm). B shows a figure in which Lgr6-positive cells prepared as in FIG. 9A were mass-produced using an EZsphere 10 cm dish from AGC Techno Glass Co., Ltd., and the section of the cell aggregate formed after transplantation into subcutaneous tissue of the back for 20 days is shown. The red blood cells of the host mouse (stained with Thermo Scientific's rat anti-mouse Ter-119 antibody, type: 12-5921-82) are present surrounding the transplanted human cells (stained with Chemicon's mouse antibody, type: MAB1281), which indicates that engraftment has occurred due to circulation from the host (scale bar is left 50 µm, right 10 µm). C shows that Lgr6 reporter GFP exists in transplanted human cells (hNA-positive cells) from left to right (scale bar = 100 µm), and hard keratin KRT17 and KRT81, which constitute the nail, are coexisting nearby (scale bar = 100 µm).

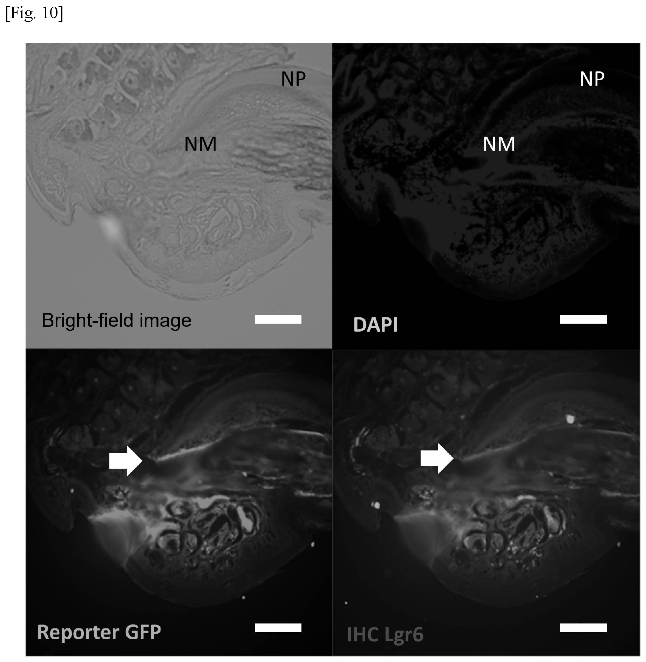

[FIG. 10] Transplantation of Lgr6-expressing cell aggregates into subcutaneous tissue of fingertips and presence in the nail matrix region. A figure showing a histological section after transplanting approximately 300 Lgr6-positive cell aggregates prepared as in FIG. 9 into the fingertip of an immunodeficient mouse, and observing it 20 days later. Cells stained with Santa Cruz's catalog number: SC-393010 antibody for Lgr6 reporter GFP are coexisting, and surviving in the fingertip tissue and nail matrix region (NM) indicated by a white arrow (scale bar = 200 µm). The nail is shown as NP (nail plate).

### [DESCRIPTION OF EMBODIMENTS]

### 1. Method for producing nail matrix cells

The present invention provides a method for producing nail matrix cells from nail stem cells (hereinafter referred to as "the production method of the present invention"). Specifically, the present invention provides a method for producing a nail matrix cell, comprising a step of culturing a nail stem cell by shaking under suspension culture conditions in the presence of a Wnt signaling activator, an FGF signaling activator, and an adrenocortical hormone.

In this specification, unless otherwise specified, the term "cell" includes a "cell population." Also, "cell population" includes "cell aggregate" and "organoid." Furthermore, unless otherwise specified, the term "cell" refers to a cell obtained by cell culture. The cell population may be constituted by one type of cells, or two or more types of cells.

Nail matrix cells are cells derived from nail stem cells *in vivo*, which divide asymmetrically and actively, and their daughter cells gradually differentiate and keratinize to form mature nails. In this specification, the term "nail matrix cell" refers to a cell that expresses Lgr6 and has the capacity of differentiating into nail cells expressing hard keratin (for example, keratin 17 (Krt-17), keratin 81 (Krt-81)). Also, in this specification, the term "nail stem cell" refers to a stem cell that expresses Lgr5 and has the capacity of differentiating into nail matrix cells.

The nail stem cells used in the present invention may, for example, utilize already established cell lines, or may be obtained from tissue of a living organism (e.g., finger tissue) or from a limb bud organoid as described below, and selected using methods such as flow cytometry or mass cytometry, magnetic cell separation, or affinity columns immobilized with the desired antigen, based on cell markers such as Lgr5.

In the present invention, the nail stem cells may be used in any of the following forms: single cells, a cell population including said cells, and a cell aggregate or organoid (e.g., limb bud organoid) containing said cells; however, they are preferably used in the form of limb bud organoid. Also, the nail matrix cells obtained by the production method of the present invention may be used in any of the following forms: single cells, a cell population including said cells, and a cell aggregate or organoid (e.g., digit organoid) containing said cells; however, they are preferably used in the form of limb bud organoid. Therefore, in another embodiment, a method for producing a nail matrix cell or a digit organoid including the cell is provided, comprising culturing a limb bud organoid by shaking under suspension culture conditions in the presence of a Wnt signaling activator, an FGF signaling activator, and an adrenocortical hormone.

In this specification, the term "organoid" refers to a structure comprising multiple types of cells, which typically has a structure and/or function similar to that of tissue in the body. Whether or not a given structure is an organoid can be determined by, for example, confirming the localization of cells and the presence or absence of layered structure formation through microscopic observation of samples subjected to dyeing (e.g., immunostaining, hematoxylineosin (HE) staining), if necessary.

In this specification, the term "digit organoid" refers to an organoid containing a nail matrix cell, and typically contains furthermore a nail cell. In one embodiment, the "digit organoid" of the present invention has Krt-81 expressing nail cells and Krt-17 expressing nail cells located proximally to Lgr6 expressing nail matrix cells. Also, the digit organoid, in addition to Lgr6 expressing nail matrix cells, Krt-81 expressing nail cells, and Krt-17 expressing nail cells, may contain at least one (preferably all) of the following: a cell expressing the Hoxd13 gene, which is essential for the development of fingers and wrists; a cell expressing the Tbox5 gene, which is specific to the forelimb; a cell expressing the pitx1 gene, which is specific to the hind limb; a cell expressing the Runx2 gene, which is involved in bone formation; and a cell expressing Lgr5. The digit organoid may express Lgr6 and/or Lgr5, and/or at least one (preferably all) of Hox genes selected from the group consisting of Hoxa11, Hoxb7, Hoxd12, and Hoxd13. Also, the digit organoid may express at least one (preferably all) of morphology-related genes selected from the group consisting of BMP2, BMP4, BMP7, FGF8, FGF18, FGF19, TGFB1, and WNT5A, and/or at least one (preferably all) of keratin genes selected from the group consisting of Krt-6a, Krt-6c, Krt-16, Krt-23, and Krt-31, and may not express at least one (preferably all) of undifferentiated state-related genes selected from the group consisting of LIN28A, POU5F1, and ESRG. In one embodiment, the digit organoid of the present invention exhibits the gene expression pattern shown in Fig. 8, but the digit organoid of the present invention is not limited to those exhibiting these gene expression patterns.

The limb bud is a primordium of vertebrate limbs that forms early in embryonic development from a part of the lateral plate mesoderm, and is formed by single-layer epithelial cells overgrowing the mesenchymal tissue. At the boundary region of the dorsal-ventral axis at the tip of the limb bud, there are an apical ectodermal ridge (AER) and a zone of polarizing activity (ZPA) on the posterior side, which function as signaling centers for limb elongation. In this specification, the term "limb bud organoid" refers to an organoid having a region containing lateral plate mesodermal cells and an E-cadherin expressing epithelial tissue surrounding the region. Examples of the lateral plate mesodermal cells include Hand2- and/or Pitx1- expressing cells. Also, the limb bud organoid typically contains nail stem cells expressing Lgr5.

In this specification, the term "Wnt signaling activator" refers to a substance that activates a signal transduction pathway in which Wnt family proteins are used as ligands and primarily Frizzled is used as a receptor. The signaling pathways include, for example, the classical Wnt pathway (canonical Wnt pathway) and non-classical Wnt pathways. The classical Wnt pathway is transmitted by β-catenin. Non-classical Wnt pathways include Planar Cell Polarity (PCP) pathway, Wnt/JNK pathway, Wnt/Calcium pathway, Wnt-RAP1 pathway, Wnt-Ror2 pathway, Wnt-PKA pathway, Wnt-GSK3MT pathway, Wnt-aPKC pathway, Wnt-RYK pathway, and Wnt-mTOR pathway. In non-classical Wnt pathways, there are common signaling factors that are activated by other signal transduction pathways besides Wnt, but activators for these factors are also included in Wnt signal transduction modulating substances. As the Wnt signaling activator, for example, GSK3 inhibitors and WNT proteins can be cited. These substances may be used alone or in combination.

GSK3 inhibitors include, for example, CHIR98014 (N-6-[2-[[4-(2,4-dichlorophenyl)-5-(1H-imidazol-1-yl)-2-pyrimidinyl]amino]ethyl]-3-nitro-2,6-pyridinediamine), CHIR99021 (6-[[2-[[4-(2,4-dichlorophenyl)-5-(5-methyl-1H-imidazol-2-yl)-2-pyrimidinyl]amino]ethyl]amino]-3-pyridinecarbonitrile), 1-Azakenpaullone (9-bromo-7,12-dihydro-pyrido[3',2':2,3]azepino[4,5-b]indol-6(5H)-one), AZD 2858 (3-amino-6-[4-[(4-methyl-1-piperazinyl)sulfonyl]phenyl]-N-3-pyridinyl-2-pyrazinecarboxamide), BIO (6-bromo-3-[(3E)-1,3-dihydro-3-(hydroxyimino)-2H-indol-2-ylidene]-1,3-dihydro-(3Z)-2H-indol-2-one), CP21R7 (3-(3-aminophenyl)-4-(1-methyl-1H-indol-3-yl)-1H-pyrrole-2,5-dione), Sotrastaurin (3-(1H-indol-3-yl)-4-[2-(4-methyl-1-piperazinyl)-4-quinazolinyl]-1H-pyrrole-2,5-dione), TWS119 (3-[[6-(3-aminophenyl)-7H-pyrrolo[2,3-d]pyrimidin-4-yl]oxy]-phenol), Valproic Acid (2-propyl-pentanoic acid), and the like. CHIR98014 is preferred. Wnt proteins include, for example, Wnt1, Wnt2, Wnt2B, Wnt3, Wnt3A, Wnt4, Wnt5A, Wnt5B, Wnt6, Wnt7A, Wnt7B, Wnt8A, Wnt8B, Wnt9A, Wnt9B, Wnt10A, Wnt10B, Wnt11, and Wnt16. Wnt3 is preferred.

The concentration of the Wnt signaling activator in the medium can be appropriately set depending on the substance used. For example, when CHIR98014, which is a type of GSK3 inhibitor, is used as the Wnt signaling activator, its concentration is usually 0.1 µM to 100 µM, preferably 1 µM to 20 µM, and more preferably 4 µM to 10 µM (in one embodiment, 7 µM). When a Wnt signaling activator other than CHIR98014 is used, the concentration of the Wnt signaling activator in the medium is appropriately selected.

An FGF signaling activator is not particularly limited as long as it can enhance the signaling pathway mediated by FGF (fibroblast growth factor). Examples of FGF signaling activators include FGF proteins such as FGF1, FGF2 (also known as bFGF), FGF3, FGF8, and FGF10, anti-FGF receptor antibodies, and FGF partial peptides. These substances may be used alone or in combination.

The FGF signaling activator is preferably at least one selected from the group consisting of FGF2, FGF3, FGF8, and FGF10, and their modified forms, and more preferably at least one (preferably both) of FGF2 and FGF8.

The concentration of the FGF signaling activator in the medium can be appropriately set depending on the substance used. For example, when FGF2 is used as the FGF signaling activator, it is usually 10 pg/ml to 1 µg/ml, preferably 100 pg/ml to 100 ng/ml, and more preferably 1 ng/ml to 20 ng/ml (in one embodiment, 10 ng/ml). When FGF8 is used, it is usually 10 pg/ml to 1 µg/ml, preferably 100 pg/ml to 100 ng/ml, and more preferably 1 ng/ml to 20 ng/ml (in one embodiment, 10 ng/ml). When FGF other than FGF2 and FGF8 is used, the concentration of FGF in the medium is appropriately selected.

Adrenocortical hormones are hormones produced by the adrenal cortex. Examples of adrenocortical hormones used in the present invention include natural glucocorticoids such as hydrocortisone, cortisone acetate, and fludrocortisone acetate, and synthetic glucocorticoids such as dexamethasone, betamethasone, prednisolone, methylprednisolone, and triamcinolone. Hydrocortisone is preferred. These substances may be used alone or in combination.

The concentration of the adrenocortical hormone in the medium can be appropriately set depending on the substance used. For example, in the case of hydrocortisone, it is usually 100 ng/ml to 1000 µg/ml, preferably 500 ng/ml to 100 µg/ml, and more preferably 1 µg/ml to 10 µg/ml (in one embodiment, 4 µg/ml).

In the production method of the present invention, nail stem cells or a limb bud organoid are cultured under suspension culture conditions. In this specification, the term "suspension culture" refers to a culture conducted under conditions that maintain cells or a cell aggregate in a suspended state in a culture medium, i.e., a culture without forming strong cell-substratum junctions between the cells or cell aggregate and the culture vessel.

The culture vessel used for suspension culture is not particularly limited, but examples include flasks, tissue culture flasks, dishes, Petri dishes, tissue culture dishes, multi-dishes, microplates, microwell plates, micropores, multiplates, multiwell plates, chamber slides, petri dishes, tubes, trays, culture bags, and roller bottles. However, for the shaking culture described later, it is preferable to use a sealed culture vessel. Examples of such culture vessels include tissue culture flasks, culture bags, and roller bottles. In addition, in order to enable culture under non-adhesive conditions, it is preferable that the culture vessel has cell non-adhesive properties. As a culture vessel with cell non-adhesive properties, one in which the surface of the culture vessel is artificially treated to be cell non-adhesive or one that is not artificially treated for the purpose of improving adhesion to cells (e.g., coating treatment with extracellular matrix) can be used.

As shown in the examples described later, digit organoids could be induced by culturing a limb bud organoid using a swirling culture method. Although it does not depend on any theory, it is presumed that the physical agitation of the medium by swirling culture enables efficient supply of oxygen and nutrients and exchange with excreted waste products, thereby inducing nail matrix cells and digit organoids. Therefore, as long as the medium can be physically agitated, the shaking culture used in the present invention is not limited to swirling culture, but may also include, for example, stirring culture. When performing shaking culture, for example, the culture vessel containing the nail stem cells or a limb bud organoid can be placed on a shaker or the like. In addition, when performing swirling culture, for example, the culture vessel containing the nail stem cells or a limb bud organoid can be placed on a rotator or the like. Furthermore, when performing stirring culture, for example, the limb bud organoid can be placed in an environment where a stirrer or the like is rotating.

The parameters such as the speed of shaking culture can be appropriately set by those skilled in the art within a range that does not cause damage to the nail stem cells or a limb bud organoid. For example, when performing shaking culture using a 3D shaker with a wave-like motion (e.g., Mini-Shaker 3D, Biosan), the shaking speed can be set in the range of, for example, 5 to 60 rpm. When performing shaking culture using a reciprocating shaker (e.g., NS-LR, Azwell Co.), the shaking speed can be set in the range of, for example, 15 to 60 rpm. When performing shaking culture using a seesaw shaker (e.g., NS-S, Azwell Co.), the shaking speed can be set in the range of, for example, 5 to 50 rpm. In the case of stirring culture, for example, a spinner flask (e.g., 3152, Corning) can be placed on a magnetic stirrer and cultured at a rotation speed such that the nail stem cells or a limb bud organoid do not visibly settle. In the case of swirling culture, culture can also be performed using a three-dimensional rotating suspension culture apparatus (e.g., CellPet CUBE, J-Tech; Clinostar, Celvivo). When performing swirling culture using a three-dimensional rotating suspension culture apparatus, the swirling speed can be set in the range of, for example, 15 to 60 rpm. In addition, when performing swirling culture using a three-dimensional rotating suspension culture apparatus, it may be swirled perpendicularly (lateral swirling) or parallel (longitudinal swirling) with respect to the gravitational direction, but lateral swirling is preferred. From the viewpoint of suppressing physical damage such as friction on cells, shaking culture of nail stem cells or a limb bud organoid embedded in a gel is also preferable.

The period for shaking culture under suspension culture conditions is not particularly limited as long as it is a period during which Lgr6-positive cells appear, but is usually 10 days or more, preferably 20 days or more, more preferably 30 days or more, even more preferably 40 days or more, and most preferably 50 days or more, and culture may be performed for 60 days or more. In addition, since nail matrix cells or a digit organoid can be maintained by culture under the above-mentioned conditions, the upper limit of the culture period is not particularly limited, but is usually 100 days or less, preferably 90 days or less, more preferably 80 days or less, and most preferably 70 days or less.

The medium used in the present invention can be prepared using a medium for culturing mammalian cells as a base medium. Examples of the base medium include BME medium, BGJb medium, CMRL 1066 medium, Glasgow MEM medium, Improved MEM Zinc Option medium, IMDM medium, Medium 199 medium, Eagle MEM medium, αMEM medium, DMEM medium, Ham's F-12 medium, RPMI1640 medium, Fischer's medium, Neurobasal medium, Essential 8 (manufactured by Thermo Fisher Scientific), Essential 6 (manufactured by Thermo Fisher Scientific), S-medium (manufactured by DS Pharma Biomedical Co., Ltd.), StemPro (manufactured by Thermo Fisher Scientific), hESF9, mTeSR1 (manufactured by STEMCELL Technologies), mTeSR2 (manufactured by STEMCELL Technologies), TeSR-E8 (manufactured by STEMCELL Technologies), mTeSR Plus (manufactured by STEMCELL Technologies), StemFit AK02N (manufactured by Ajinomoto Co., Inc.), StemFit AK03N (manufactured by Ajinomoto Co., Inc.), ReproMed iPSC Medium (manufactured by ReproCeller, Inc.), NutriStem XF (manufactured by Biological Industries), NutriStem V9 (manufactured by Biological Industries), Cellartis DEF-CS Xeno-Free Culture Medium (manufactured by Takara Bio), Stem-Partner SF (manufactured by Kyodo Yakuhin Co., Ltd.), PluriSTEM Human ES/iPS Cell Medium (manufactured by Merck), and StemSure hPSC MediumΔ (manufactured by Fujifilm Wako Pure Chemical Corporation), as well as mixtures thereof (e.g., DMEM/F-12 medium (a mixture of DMEM medium and Ham's F-12 medium in a 1:1 ratio)). In one embodiment, a mixture of DMEM/F-12 medium and Neurobasal medium is used. The mixing ratio, by volume, is, for example, DMEM/F-12 : Neurobasal = 0.8 to 1.2 : 1.2 to 0.8 (preferably 1:1).

The medium used in the present invention may be a serum-containing medium or a serum-free medium. A serum-free medium refers to a medium that does not contain unprocessed or unpurified serum, and a medium containing purified blood-derived components or animal tissue-derived components (e.g., growth factors) shall be considered as a serum-free medium. The medium used in the present invention may also contain a serum substitute. Serum substitutes can include, for example, albumin, transferrin, fatty acids, collagen precursors, trace elements, 2-mercaptoethanol or 3'-thiolglycerol, or equivalents thereof. Such serum substitutes can be prepared by the method described in WO98/30679. In addition, to simplify the implementation of the production method of the present invention, commercially available serum substitutes can be used. Examples of such commercially available serum substitutes include KSR (knockout serum replacement) (manufactured by Invitrogen), Chemically-defined Lipid concentrated (manufactured by Gibco), and Glutamax (manufactured by Gibco).

The medium may contain other additives. Additives can include, for example, insulin, an iron source (e.g., transferrin), minerals (e.g., sodium selenite), sugars (e.g., glucose), organic acids (e.g., pyruvic acid, lactic acid), serum proteins (e.g., albumin), amino acids (e.g., L-glutamine), reducing agents (e.g., 2-mercaptoethanol), vitamins (e.g., ascorbic acid, d-biotin), antibiotics (e.g., streptomycin, penicillin, gentamicin), and buffers (e.g., HEPES), but are not limited thereto.

The culture temperature is not particularly limited, but is usually 30°C to 40°C, preferably 37°C, and the culture is carried out in an atmosphere of CO₂-containing air, with a CO₂ concentration of preferably 2% to 5%.

The limb bud organoid used in the present invention can be prepared by differentiating from pluripotent stem cells or the like. Such methods include, for example, methods including (A) to (D) below. Therefore, the production method of the present invention may include at least one of steps (A) to (D).
(A) A step of culturing a pluripotent stem cell in suspension to form a cell aggregate of the pluripotent stem cells.
(B) A step of culturing the cell aggregate obtained in step (A) in suspension in the presence of a BMP signaling activator to induce the cell aggregate.
(C) A step of culturing the cell aggregate obtained in step (B) in suspension in the presence of a BMP signaling activator and a retinoic acid signaling activator to induce the cell aggregate.
(D) A step of culturing the cell aggregate obtained in step (C) in suspension in the presence of a BMP signaling inhibitor to induce a limb bud organoid.

Any pluripotent stem cells may be used for the present invention as long as they are undifferentiated cells that have "self-renewal capacity" in which they can be grown while maintaining an undifferentiated state and "pluripotency" in which they can be differentiated into all the three primary germ layers. Examples of such pluripotent stem cells include induced pluripotent stem cells (iPS cells), embryonic stem cells (ES cells), cloned embryo-derived nuclear transfer embryonic stem cells (ntES cells), multipotent germline stem cells ("mGS cells"), and embryonic germline stem cells (EG cells), with iPS cells being preferred (and human iPS cells being more preferred). When the pluripotent stem cells are ES cells or any human embryo-derived cells, the cells may be cells prepared with embryo destruction or cells prepared without embryo destruction, but are preferably cells prepared without embryo destruction from an ethical point of view.

iPS cells are somatic cell-derived induced stem cells that can be prepared by introducing a particular reprogramming factor into somatic cells in the form of a DNA or protein, and have properties almost comparable to those of ES cells, such as pluripotency and self-renewal proliferative capacity (Takahashi K. and Yamanaka S. (2006) Cell, 126:663-676; Takahashi K. et al. (2007), Cell, 131:861-872; Yu J. et al. (2007), Science, 318:1917-1920; Nakagawa M. et al., Nat. Biotechnol. 26:101-106 (2008); WO 2007/069666). When iPS cells are used, the iPS cells may be prepared from somatic cells by a method known per se, or may have already been established and stocked. The reprogramming factor may be constituted by a gene specifically expressed in ES cells, or a gene product or non-coding RNA thereof, or a gene playing an important role in maintaining the undifferentiated state of ES cells, or a gene product or non-coding RNA thereof, or a small molecule. Examples of the gene contained in the reprogramming factor include Oct3/4, Sox2, Sox1, Sox3, Sox15, Sox17, Klf4, Klf2, c-Myc, N-Myc, L-Myc, Nanog, Lin28, Fbx15, ERas, ECAT15-2, Tcl1, beta-catenin, Lin28b, Sall1, Sall4, Esrrb, Nr5a2, Tbx3, and Glis1. Such reprogramming factors may be used alone or in combination. Examples of combinations of the reprogramming factors include those described in WO 2007/069666, WO 2008/118820, WO 2009/007852, WO 2009/032194, WO 2009/058413, WO 2009/057831, WO 2009/075119, WO 2009/079007, WO 2009/091659, WO 2009/101084, WO 2009/101407, WO 2009/102983, WO 2009/114949, WO 2009/117439, WO 2009/126250, WO 2009/126251, WO 2009/126655, WO 2009/157593, WO 2010/009015, WO 2010/033906, WO 2010/033920, WO 2010/042800, WO 2010/050626, WO 2010/056831, WO 2010/068955, WO 2010/098419, WO 2010/102267, WO 2010/111409, WO 2010/111422, WO 2010/115050, WO 2010/124290, WO 2010/147395, WO 2010/147612, Huangfu D, et al. (2008), Nat. Biotechnol., 26:795-797, Shi Y, et al. (2008), Cell Stem Cell, 2:525-528, Eminli S, et al. (2008), Stem Cells. 26:2467-2474, Huangfu D, et al. (2008), Nat Biotechnol. 26:1269-1275, Shi Y, et al. (2008), Cell Stem Cell, 3, 568-574, Zhao Y, et al. (2008), Cell Stem Cell, 3:475-479, Marson A, (2008), Cell Stem Cell, 3, 132-135, Feng B, et al. (2009), Nat Cell Biol. 11:197-203, R.L. Judson et al., (2009), Nat. Biotech., 27:459-461, Lyssiotis CA, et al. (2009), Proc Natl Acad Sci U S A. 106:8912-8917, Kim JB, et al. (2009), Nature. 461:649-643, Ichida JK, et al. (2009), Cell Stem Cell. 5:491-503, Heng JC, et al. (2010), Cell Stem Cell. 6:167-74, Han J, et al. (2010), Nature. 463:1096-100, Mali P, et al. (2010), Stem Cells. 28:713-720, and Maekawa M, et al. (2011), Nature. 474:225-9.

ES cells are stem cells that are established from an inner cell mass of the early embryo (such as blastocyst) of mammals such as humans and mice and have pluripotency and self-renewal proliferative capacity. ES cells have been discovered in mice in 1981 (M.J. Evans and M.H. Kaufman (1981), Nature 292:154-156), and then ES cell lines have also been established in primates such as humans and monkeys (J.A. Thomson et al. (1998), Science 282:1145-1147; J.A. Thomson et al. (1995), Proc. Natl. Acad. Sci. USA, 92:7844-7848; J.A. Thomson et al. (1996), Biol. Reprod., 55:254-259; J.A. Thomson and V.S. Marshall (1998), Curr. Top. Dev. Biol., 38:133-165). ES cells can be established by removing an inner cell mass from the blastocyst of a fertilized egg of an animal of interest and culturing the inner cell mass on fibroblast feeders. Methods for establishing and maintaining human and monkey ES cells are described in US Patent No. 5,843,780; Thomson JA, et al. (1995), Proc Natl. Acad. Sci. U S A. 92:7844-7848; Thomson JA, et al. (1998), Science. 282:1145-1147; Suemori H. et al. (2006), Biochem. Biophys. Res. Commun., 345:926-932; Ueno M. et al. (2006), Proc. Natl. Acad. Sci. USA, 103:9554-9559; Suemori H. et al. (2001), Dev. Dyn., 222:273-279; Kawasaki H. et al. (2002), Proc. Natl. Acad. Sci. USA, 99:1580-1585; and Klimanskaya I. et al. (2006), Nature. 444:481-485, for example. Alternatively, ES cells can be established using only single blastomeres of embryos in the cleavage stage prior to the blastocyst stage (Chung Y. et al. (2008), Cell Stem Cell 2: 113-117), or can be established using arrested embryos (Zhang X. et al. (2006), Stem Cells 24: 2669-2676).

ntES cells are cloned embryo-derived ES cells prepared by a nuclear transfer technique, and have properties almost comparable to those of fertilized egg-derived ES cells (Wakayama T. et al. (2001), Science, 292:740-743; S. Wakayama et al. (2005), Biol. Reprod., 72:932-936; Byrne J. et al. (2007), Nature, 450:497-502). That is, ntES (nuclear transfer ES) cells are ES cells established from an inner cell mass of the cloned embryo-derived blastocyst obtained by replacing the nucleus of an unfertilized egg with the nucleus of somatic cells. For preparation of ntES cells, a combination of a nuclear transfer technique (Cibelli J.B. et al. (1998), Nature Biotechnol., 16:642-646) with an ES cell preparation technique (supra.) is utilized (Sayaka Wakayama et al. (2008), Jikken Igaku [Experimental Medicine], Vol. 26, No. 5 (suppl.), pp. 47-52). In nuclear transfer, reprogramming can be performed by injecting the nucleus of somatic cells into an enucleated unfertilized egg of a mammal and culturing for several hours.

mGS cells are testis-derived pluripotent stem cells and are cells of spermatogenic origin. The cells can be differentiated into various lineages of cells by induction as in ES cells, and can produce chimeric mice when they are transplanted into the mouse blastocyst, for example (Kanatsu-Shinohara M. et al. (2003) Biol. Reprod., 69:612-616; Shinohara K. et al. (2004), Cell, 119:1001-1012). The cells are capable of self-renewal in a culture solution containing a glial cell line-derived neurotrophic factor (GDNF), and can be repeatedly passaged under the same culture conditions as for ES cells to obtain germline stem cells (Masanori Takebayashi et al. (2008), Jikken Igaku [Experimental Medicine], Vol. 26, No. 5 (suppl.), pp. 41-46, Yodosha (Tokyo, Japan)).

EG cells are cells that are established from primordial germ cells in the embryonic stage and have the same pluripotency as in ES cells. The cells can be established by culturing primordial germ cells in the presence of a substance such as LIF, bFGF, or a stem cell factor (Matsui Y. et al. (1992), Cell, 70:841-847; J.L. Resnick et al. (1992), Nature, 359:550-551).

The species from which the pluripotent stem cells are derived are not particularly limited, and may be rodents such as rats, mice, hamsters, and guinea pigs; Lagomorpha such as rabbits; Ungulata such as pigs, cows, goats, and sheep; Carnivora such as dogs and cats; and primates such as humans, monkeys, rhesus monkeys, marmosets, orangutans, and chimpanzees, for example. The species is preferably humans.

In step (A), there is no particular limitation on the method for preparing a multicellular aggregate of pluripotent stem cells. In one preferred embodiment, the multicellular aggregate of pluripotent stem cells can be obtained by dispersing pluripotent stem cells and culturing them under suspension culture conditions to allow multiple pluripotent stem cells to assemble and form an aggregate.

The formation of the multicellular aggregate of pluripotent stem cells in step (A) is typically performed by the following method. First, pluripotent stem cells are collected from a passage culture and dispersed into single cells or a state close thereto. The dispersion of pluripotent stem cells is performed using an appropriate cell dissociation solution. As the cell dissociation solution, for example, EDTA; trypsin, collagenase IV, metalloprotease, or other protein-degrading enzymes can be used alone or in combination as appropriate. Among these, those with low cytotoxicity are preferred, and commercially available products such as Dispase (Eidia), TrypLE (Invitrogen), or Accutase (MILLIPORE) can be used as such cell dissociation solutions. The dispersed pluripotent stem cells are suspended in the above-mentioned medium.

In order to suppress cell death of pluripotent stem cells (particularly human pluripotent stem cells) induced by dispersion, it is preferable to add an inhibitor of Rho-associated coiled-coil kinase (ROCK) from the start of culture (JP Publication No. 2008-99662). As the ROCK inhibitor, Y-27632 ((+)-(R)-trans-4-(1-aminoethyl)-N-(4-pyridyl)cyclohexanecarboxamide dihydrochloride), fasudil/HA1077, SR3677, GSK269962, GSK429286A, H1152, Wf-536, thiazovivin and their salts or derivatives can be mentioned. The concentration of the ROCK inhibitor used in suspension culture is a concentration that can suppress cell death of pluripotent stem cells induced by dispersion. For example, for Y-27632, such a concentration is usually 0.1 to 200 µM, preferably 2 to 50 µM (in one embodiment, 10 µM). The treatment with the ROCK inhibitor is typically carried out for one day, and thereafter, it is preferable to continue culturing in a medium not containing the ROCK inhibitor.

In step (A) and any of the subsequent steps, when a medium exchange operation is performed, for example, an operation of adding new medium without discarding the original medium (medium addition operation), an operation of discarding about half of the original medium (about 30 to 90% of the volume of the original medium, for example, about 40 to 60%) and adding about half of the new medium (30 to 90% of the volume of the original medium, for example, about 40 to 60%) (half-volume medium exchange operation), or an operation of discarding about all of the original medium (90% or more of the volume of the original medium) and adding about all of the new medium (90% or more of the volume of the original medium) (full-volume medium exchange operation) can be mentioned.

As the basal medium used in step (A), the above-mentioned basal media that can be used in the production method of the present invention can be mentioned, but preferably StemFit AK02N medium or Essential 8 medium. Essential 8 medium is a medium containing L-ascorbic acid-2-phosphate magnesium (64 mg/l), sodium selenium (14 µg/l), insulin (19.4 mg/l), NaHCO₃ (543 mg/l), transferrin (10.7 mg/l), bFGF (100 ng/mL), and a TGFβ family signaling activator (TGFβ1 (2 ng/mL) or Nodal (100 ng/mL)) as additives in DMEM/F12 medium (Nature Methods, 8, 424-429 (2011)). It is also preferable to change the basal medium during culture. For example, StemFit AK02N medium may be used at the start of culture, and Essential 8 medium may be used for the last day of culture. The medium used in step (A) may contain the above-mentioned medium additives that can be used in the production method of the present invention.

The duration of step (A) can be appropriately set by those skilled in the art, and is usually 1 day to 10 days, preferably 2 days to 8 days, and more preferably 3 days to 7 days (in one embodiment, 5 days).

In step (B), the cell aggregate obtained in step (A) is cultured under suspension culture conditions in the presence of a BMP signaling activator, thereby inducing the cell aggregate. In one embodiment, the cell aggregate obtained in step (B) is a cell aggregate containing posterior primitive streak (PPS) cells.

The BMP signaling activator used in step (B) is a substance that can enhance the signaling pathway mediated by bone morphogenetic protein (BMP). Substances that can enhance the signaling pathway mediated by BMP include, for example, substances that stabilize and improve the titer of BMP ligands in the culture environment, substances that bind to ALK-1, ALK-2, ALK-3, and ALK-6, which are type I BMP receptors, and induce downstream intracellular signal transduction, substances that induce phosphorylation of Smad-1, Smad-5, Smad-8, and Smad-9 involved in intracellular BMP signaling, and substances that induce or enhance functions such as activation or suppression of gene transcription by Smad-1/5/8/9. As the BMP signaling activator, for example, proteins such as BMP2, BMP4, BMP7, BMP13, GDF7 (among these, BMP2, BMP4, and BMP7 are preferred, and particularly BMP4 is preferred), GDF proteins such as GDF5, 6, 7, anti-BMP receptor antibodies, and BMP partial peptides can be mentioned. These substances may be used alone or in combination.

The concentration of the BMP signaling activator in the medium can be appropriately set according to the substance used. For example, when BMP4 is used as the BMP signaling activator, it is usually used at a concentration of 1 ng/ml to 200 ng/ml, preferably 5 ng/ml to 40 ng/ml, and more preferably 10 ng/ml to 20 ng/ml. When a BMP signaling activator other than BMP4 is used, the concentration of the BMP signaling activator in the medium is appropriately selected.

As the BMP signaling activator, compounds known to those skilled in the art can also be used. Examples of BMP signaling activators include Smurf1 inhibitors, Chk1 inhibitors, and phosphorylated Smad stabilizers. Compounds having the above-mentioned activity include, for example, A-01 ([4-[[4-Chloro-3-(trifluoromethyl)phenyl]sulfonyl]-1-piperazinyl][4-(5-methyl-1H-pyrazol-1-yl)phenyl]methanone), PD407824 (9-Hydroxy-4-phenyl-pyrrolo[3,4-c]carbazole-1,3(2H,6H)-dione), SB4 (2-[[(4-Bromophenyl)methyl]thio]benzoxazole), SJ000291942 (2-(4-Ethylphenoxy)-N-(4-fluoro-3-nitrophenyl)-acetamide), and derivatives thereof.

The basal medium used in step (B) can be the same as the above-mentioned media that can be used in the production method of the present invention. Essential 6 medium and DMEM medium are preferred as the basal medium. It is also preferable to change the basal medium during the culture, for example, by using Essential 6 medium at the start of the culture and DMEM medium for the last day of the culture. The medium used in step (B) may contain the above-mentioned medium additives that can be used in the production method of the present invention.

The culture period in step (B) can be appropriately set by those skilled in the art, and is usually 4 days to 14 days, preferably 5 days to 13 days, and more preferably 6 days to 12 days (in one embodiment, 9 days).

In one embodiment, the medium used in step (B) (particularly, the culture in the latter half of step (B)) may further contain at least one (preferably all) selected from the group consisting of insulin, transferrin, and sodium selenite. The concentration of insulin in the medium is typically 0.1 to 100 µg/mL, preferably 1 to 50 µg/mL, and more preferably 3 to 20 µg/mL (particularly 10 µg/mL). The concentration of transferrin in the medium is typically 0.1 to 100 µg/mL, preferably 1 to 50 µg/mL, and more preferably 2 to 10 µg/mL (particularly 5.5 µg/mL). The concentration of sodium selenite in the medium is typically 0.1 to 100 pg/mL, preferably 1 to 50 pg/mL, and more preferably 2 to 10 pg/mL (particularly 6.7 pg/mL).

In step (C), the cell aggregate obtained in step (B) is suspended and cultured in the presence of a BMP signaling activator and a retinoic acid signaling activator, thereby inducing the cell aggregates. The BMP signaling activator may be the same as that used in step (B). In one embodiment, the cell aggregate obtained in step (C) is a cell aggregate containing lateral plate mesoderm (LPM) cells.

Retinoic acid signaling activators include, for example, substances that bind to retinoic acid receptors (RAR) or retinoid X receptors (RXR) and activate downstream transcription. Compounds having the above-mentioned effects include, for example, all-trans retinoic acid, isotretinoin, 9-cis retinoic acid, TTNPB (4-[(E)-2-[(5,5,8,8-Tetramethyl-5,6,7,8-tetrahydronaphthalene)-2-yl]-1-propenyl]benzoic acid), Ch55 (4-[(E)-3-(3,5-di-tert-butylphenyl)-3-oxo-1-propenyl]benzoic acid), EC19 (3-[2-(5,6,7,8-Tetrahydro-5,5,8,8-tetramethyl-2-naphthalenyl)ethynyl]benzoic acid), EC23 (4-[2-(5,6,7,8-Tetrahydro-5,5,8,8-tetramethyl-2-naphthalenyl)ethynyl)-benzoic acid), Fenretinide (4-hydroxyphenylretinamide), Acitretin ((all-e)-9-(4-methoxy-2,3,6-trimethylphenyl)-3,7-dimethyl-2,4,6,8-nonatetraen), Trifarotene, Adapalene, AC 261066 (4-[4-(2-Butoxyethoxy)-5-methyl-2-thiazolyl]-2-fluorobenzoic acid), AC 55649 (4-N-Octylbiphenyl-4-carboxylic acid), AM 580 (4-[(5,6,7,8-Tetrahydro-5,5,8,8-tetramethyl-2-naphthalenyl)carboxamido]benzoic acid), AM80 (4-[(5,5,8,8-Tetramethyl-6,7-dihydronaphthalen-2-yl)carbamoyl]benzoic acid), BMS 753 (4-[[(2,3-Dihydro-1,1,3,3-tetramethyl-2-oxo-1H-inden-5-yl)carbonyl]amino]benzoic acid), BMS 961 (3-Fluoro-4-[(r)-2-hydroxy-2-(5,5,8,8-tetramethyl-5,6,7,8-tetrahydro-naphthalen-2-yl)-acetylamino]-benzoic acid), CD1530 (4-(6-Hydroxy-7-tricyclo[3.3.1.13,7]dec-1-yl-2-naphthalenyl)benzoic acid), CD2314 (5-(5,6,7,8-Tetrahydro-5,5,8,8-tetramethyl-2-anthracenyl)-3-thiophenecarboxylic acid), CD437 (2-naphthalenecarboxylic acid,6-(4-hydroxy-3-tricyclo(3.3.1.1(3,7))dec-1-ylphen), CD271 (6-[3-(1-Adamantyl)-4-methoxyphenyl]-2-naphthalene carboxylic acid), and derivatives thereof. These substances may be used alone or in combination.

The retinoic acid signaling activator in step (C) is preferably all-trans retinoic acid. The concentration of the retinoic acid signaling activator in the medium can be appropriately set depending on the substance used. When all-trans retinoic acid is used as the retinoic acid signaling activator, for example, the concentration of all-trans retinoic acid is 200 nM to 20 µM, preferably 500 nM to 5 µM (in one embodiment, 2 µM). When a retinoic acid signaling activator other than all-trans retinoic acid is used, the concentration of the retinoic acid signaling activator in the medium is appropriately selected.

As the basal medium used in step (C), for example, the same medium as that used in the production method of the present invention can be used. DMEM medium is preferred as the basal medium. The medium used in step (C) may contain the above-mentioned medium additives that can be used in the production method of the present invention.

The culture period in step (C) can be appropriately set by those skilled in the art, and is usually from 1 day to 7 days, preferably from 2 days to 6 days, more preferably from 3 days to 5 days (in one embodiment, 4 days).

In one embodiment, the medium used in step (C) may further comprise at least one (preferably all) selected from the group consisting of insulin, transferrin, and sodium selenite, as in the medium of step (B). The concentrations of insulin, transferrin, and sodium selenite in the medium may be the same as those in the medium of step (B).

In step (D), the cell aggregate obtained in step (C) is suspended and cultured in the presence of a BMP signaling inhibitor, thereby inducing a limb bud organoid.

The BMP signaling inhibitor used in step (D) is not limited as long as it can suppress the signal transduction induced by BMP family proteins. It may be a nucleic acid, protein, or low molecular weight organic compound. Examples of such substances include those that inhibit BMP processing and secretion into the extracellular space, those that directly act on BMP (e.g., proteins, antibodies, aptamers), those that suppress the expression of genes encoding BMP (e.g., antisense oligonucleotides, siRNA), those that inhibit the binding of BMP receptors and BMP, and those that inhibit the physiological activity resulting from signal transduction by BMP receptors. There are type I BMP receptors and type II BMP receptors, with known type I BMP receptors including BMPR1A, BMPR1B, ACVR, and known type II BMP receptors including TGF-beta R-II, ActR-II, ActR-IIB, BMPR2, MISR-II.

Examples of proteins known as BMP signaling inhibitors include Noggin, Chordin, Follistatin, Gremlin, Inhibin, Twisted Gastrulation, Coco, and secreted proteins belonging to the DAN family. As BMP signaling inhibitors, substances that inhibit the binding of BMP receptors and BMP, and those that inhibit the physiological activity resulting from signal transduction by BMP receptors are preferred, and more preferably, an inhibitor of type I BMP receptor.

As a BMP signaling inhibitor, compounds known to those skilled in the art can also be used. As BMP signaling inhibitors, for example, inhibitors of type I BMP receptors can be mentioned. Compounds having such activity include, for example, K02288 (3-[(6-Amino-5-(3,4,5-trimethoxyphenyl)-3-pyridinyl]phenol,3-[6-Amino-5-(3,4,5-trimethoxyphenyl)-3-pyridinyl]-phenol), Dorsomorphin (6-[4-[2-(1-Piperidinyl)ethoxy]phenyl]-3-(4-pyridinyl)pyrazolo[1,5-a]pyrimidine), LDN193189 (4-[6-[4-(1-Piperazinyl)phenyl]pyrazolo[1,5-a]pyrimidin-3-yl]quinoline dihydrochloride), LDN212854 (5-[6-[4-(1-Piperazinyl)phenyl]pyrazolo[1,5-a]pyrimidin-3-yl]quinoline), LDN214117 (1-(4-(6-methyl-5-(3,4,5-trimethoxyphenyl)pyridin-3-yl)phenyl)piperazine), ML347 (5-[6-(4-Methoxyphenyl)pyrazolo[1,5-a]pyrimidin-3-yl]quinoline)), DMH1 (4-(6-(4-Isopropoxyphenyl)pyrazolo[1,5-a]pyrimidin-3-yl)quinoline), DMH2 (4-[6-[4-[2-(4-Morpholinyl)ethoxy]phenyl]pyrazolo[1,5-a]pyrimidin-3-yl]-quinoline), Compound 1 (3-(1,2,3-benzothiadiazol-6-yl)-1-[2-(cyclohex-1-en-1-yl)ethyl]urea), VU0465350 (7-(4-isopropoxyphenyl)-3-(1H-pyrazol-4-yl)imidazo[1,2-a]pyridine), VU0469381 (5-(6-(4-methoxyphenyl)pyrazolo[1,5-a]pyrimidin-3-yl)quinolone), OD36 (4-chloro-7,10-dioxa-13,17,18,21-tetrazatetracyclo[12.5.2.12,6.017,20]docosa-1(20),2(22),3,5,14(21),15,18-heptaene), OD52, E6201 ((3S,4R,5Z,8S,9S,11E)-14-(ethylamino)-8,9,16-trihydroxy-3,4-dimethyl-3,4,9,10-tetrahydro-1H-benzo[c][1]oxacyclotetradecine-1,7(8H)-dione), Saracatinib (N-(5-chloro-1,3-benzodioxol-4-yl)-7-[2-(4-methylpiperazin-1-yl)ethoxy]-5-(oxan-4-yloxy)quinazolin-4-amine), BYL719 ((2S)-1-N-[4-methyl-5-[2-(1,1,1-trifluoro-2-methylpropan-2-yl)pyridin-4-yl]-1,3-thiazol-2-yl]pyrrolidine-1,2-dicarboxamide), and the like. These substances may be used alone or in combination.

The concentration of BMP signaling inhibitors in the culture medium can be appropriately set according to the substance used. For example, when LDN193189 is used as a BMP signaling inhibitor in step (D), the concentration is typically 20 nM to 100 µM, preferably 100 nM to 50 µM, more preferably 200 nM to 10 µM, and even more preferably 500 nM to 5 µM (in one embodiment, 2 µM). When a BMP signaling inhibitor other than LDN193189 is used, it is desirable that the concentration at which the substance exhibits equivalent BMP signaling inhibitory activity to the above-mentioned concentrations of LDN193189 be employed. When a BMP signaling inhibitor other than LDN193189 is used, the concentration of the BMP signaling inhibitor in the culture medium is appropriately selected.

As the culture medium used in step (D), for example, the same culture medium as that used in the production method of the present invention can be cited. The culture medium used in step (D) may contain the above-mentioned culture medium additives that can be used in the production method of the present invention.

The culture period in step (D) can be appropriately set by those skilled in the art, and is typically 6 hours to 2 days, preferably 12 hours to 36 hours (in one embodiment, 24 hours).

In one embodiment, the culture medium used in step (D) may further contain at least one (preferably all) selected from insulin, transferrin, and sodium selenite, similar to the culture medium of step (B). The concentrations of insulin, transferrin, and sodium selenite in the culture medium may be the same as those in step (B).

The culture temperature in the above-mentioned steps (A) to (D) is not particularly limited, but is typically 30°C to 40°C, preferably 37°C. Culturing is performed under an atmosphere of CO₂-containing air, and the CO₂ concentration is preferably 2% to 5%.

The limb bud organoid used in the production method of the present invention may be obtained by methods other than those described above. As such a method, for example, the method described in Non-Patent Literature 1 can be cited. In Non-Patent Literature 1, specifically, a limb bud organoid is prepared by the following procedure: mouse embryonic stem cells maintained in maintenance medium (G-MEM added with 1% FBS, 10% KSR, 0.1 mM non-essential amino acids, 1 mM pyruvate, 0.1 mM 2-mercaptoethanol, 2000 U/ml LIF, 1 µM PD0325901, and 3 µM CHIR99021) are suspended in differentiation medium (G-MEM added with 1.5% KSR, 0.1 mM non-essential amino acids, 1 mM pyruvate, and 0.1 mM 2-mercaptoethanol) at a concentration of 3000 cells per well in cell non-adhesive plates to form a multipotent stem cell cell aggregate (SFEBq method) (day 0 of culture). From day 1 to day 5 of culture, human BMP4 and matrigel are added to the medium at final concentrations of 10 ng/ml and 4% (v/v), respectively, to induce differentiation into PPS (posterior primitive streak) aggregates. On day 5 of culture, the cells are transferred to bacterial plastic dishes, and the amnion-like membrane on the surface of the PPS aggregate is removed with tweezers. Thereafter, the differentiation-inducing factor added differs depending on whether the PPS aggregate is to be differentiated into a mouse hind limb or forelimb. When differentiation into a mouse hind limb is intended, the PPS aggregate is cultured in 2.5% KSR differentiation medium from day 5 to day 6. From day 6 onward, it is changed to LB differentiation medium (LB-medium) containing ITS + 1 and 0.1 mM 2-mercaptoethanol, and 1 µM AGN193109 (retinoic acid antagonist (RAA)) is added from day 6 to day 6.5 to induce differentiation into LPM (lateral plate mesoderm) aggregates for hind limb formation. On the other hand, when a mouse forelimb is intended, PPS aggregates cultured in 2.5% KSR differentiation medium containing 1 µM all-trans retinoic acid (RA) from day 5 to day 6 are exchanged with LB medium from day 6 to day 6.5 to induce differentiation into LPM aggregates for forelimb formation. From day 6.5 onward, both aggregates are cultured in LB medium added with 4 µg/ml hydrocortisone and 0.8% methylcellulose 40, and 0.1 µM LDN193189 is added from day 6.5 to day 7 to induce differentiation into a limb bud organoid.

### 2. Nail matrix cell producible by the production method of the present invention

In this way, nail matrix cells or a digit organoid containing said cells can be produced from nail stem cells or a limb bud organoid. Therefore, in another aspect of the present invention, a nail matrix cell or a digit organoid containing said cell producible by the production method of the present invention (hereinafter referred to as "the cell or organoid of the present invention") are provided. Furthermore, nail matrix cells can be isolated from a cell population, cell aggregate, or digit organoid containing nail matrix cells produced by the production method of the present invention by known means. Therefore, the present invention also provides a method for isolating a nail matrix cell from a cell population, cell aggregate, or digit organoid containing the nail matrix cell producible by the production method of the present invention, and a nail matrix cell isolatable by said method.

The isolation of nail matrix cells from a cell population, a cell aggregate, or a digit organoid comprising nail matrix cells can be performed by the following method. For example, after dispersing the cell aggregate such as an organoid into single-cell populations, cells expressing a marker protein present on the surface of the nail matrix cells, for example, Lgr6, are separated. Next, the above-mentioned pretreated cell population is dispersed by enzymatic treatment. Specifically, first, the above-mentioned pretreated cell population is transferred to a culture vessel containing PBS and washed with the same medium. The enzyme for dispersion is not particularly limited as long as it can disperse cells; examples include papain, EDTA, trypsin, collagenase (collagenase types I to VII), metalloprotease, hyaluronidase, elastase, dispase, deoxyribonuclease, or mixtures thereof. Papain is a preferred enzyme. The conditions for enzymatic treatment (temperature, time, etc.) can be appropriately set depending on the enzyme used.

### 3. An agent for treating fingertip disorders comprising a nail matrix cell or a digit organoid

Nail matrix cells continue to divide *in vivo*, and the divided cells keratinize and mature into nails. However, damage to the fingertips or chemotherapy with anticancer drugs can cause loss of nail matrix cells, resulting in significant impairment of nail formation. As shown in the examples described below, in one embodiment, the digit organoid of the present invention expresses the Hoxd13 gene, which is a transcription factor involved in finger formation, and the Runx2 gene, which is involved in skeletal formation. In sarcomas derived from nail matrix cells or nail stem cells, it has been reported that structures similar to fingers are reproduced (e.g., Godse R. et al., J Cutan Pathol. 2023 Sep;50(9):779-782; Lee S., Kim B.J. and Lee W., Australas J Dermatol. 2019 Feb;60(1):69-70, etc.). Furthermore, as shown in the examples described below, it has been demonstrated that nail organoids can be implanted into mice and engraft *in vivo*. Therefore, by transplanting the cell or organoid of the present invention into patients with fingertip disorders including nail disorders, regeneration of the fingertips and nails is expected, which can lead to treatment of fingertip disorders. Therefore, the present invention provides an agent for treating fingertip disorders comprising the cell or organoid of the present invention (hereinafter referred to as "the therapeutic agent of the present invention"). In one embodiment, the therapeutic agent of the present invention is a therapeutic agent for nail disorders. The nail matrix cells contained in the therapeutic agent of the present invention may be isolated from a cell population such as a digit organoid. In this specification, the term "fingertip" refers to the region from the second joint of the finger to the distal end of the finger, but typically refers to the region from the first joint of the finger to the distal end of the finger. Furthermore, the present invention also encompasses a method for treating fingertip disorders by transplanting an effective amount of nail matrix cells or a digit organoid of the present invention into a mammal (e.g., human, mouse, rat, monkey, cow, horse, pig, dog, etc.) to be treated.

The cell or organoid of the present invention can be used as an agent for treating fingertip disorders, or as a therapeutic agent for nail disorders based on damage to nail matrix cells, or to replenish the corresponding damaged site in the damaged state of the nail matrix cells. The fingertip disorder may be a congenital factor or an acquired factor. Examples of factors causing fingertip disorders include congenital malformations or deformities of limbs and fingers, loss or damage of fingertips due to accidents, necrosis or detachment of fingertips due to frostbite, infections, internal organ diseases, tumors, and side effects of drugs such as anticancer agents. Examples of nail disorders include congenital anonychia, complete or partial loss of nails due to injury, green nail syndrome, onychomycosis, acute paronychia, and nail tumors.

The therapeutic agent of the present invention comprises a pharmaceutically acceptable carrier. As the pharmaceutically acceptable carrier, there are saline, phosphate-buffered physiological saline (PBS), media, etc., for stably maintaining cells. The medium is not particularly limited, but examples include RPMI, AIM-V, and X-VIVO10, and the like. Furthermore, as a pharmaceutically acceptable carrier, preservatives, stabilizers, reducing agents, isotonicizing agents, and the like that are commonly used may be added. If necessary, cryopreservatives can be added for cryopreservation, thawed at the time of use, washed with a buffer solution, and used for transplantation therapy.

The effective amount of nail matrix cells or digit organoid contained in the therapeutic agent of the present invention is appropriately selected according to the condition, weight, age, etc., of the subject, but usually contains 1×10² to 1×10⁵ cells per dose.

The therapeutic agent of the present invention can be formulated into a known form suitable for non-oral administration, such as an injection or infusion. When used as an infusion, it may be transplanted once at the root of the nail of the subject to be treated, or transplanted multiple times.

### 4. Nail matrix cell comprising a nucleic acid encoding a reporter protein

The present invention also provides a nail matrix cell (hereinafter referred to as "the reporter cell of the present invention") having a nucleic acid (hereinafter referred to as "the nucleic acid of the present invention") comprising a reporter gene under the control of the transcriptional regulatory region of the Lgr6 gene.

In this specification, the term "transcription regulatory region" normally refers to a region ranging from several kb upstream to tens of kb upstream of a gene. For example, it can be identified by methods including: (i) determining the 5' end by conventional methods such as the 5'-RACE method (e.g., using 5'-full Race Core Kit from Takara Bio), oligo cap method, and S1 primer mapping; and (ii) acquiring a 5'-upstream region using a Genome Walker Kit (from Clontech), and measuring promoter activity of the obtained upstream region.

Human Lgr6 has three alternative splicing variants, and the transcription regulatory region of any of these variants can be used in the present invention, but preferably it is the transcription regulatory region of variant 2. The transcription regulatory region of the Lgr6 gene contained in the nucleic acid of the present invention is not limited to this, and examples include the base sequence shown in SEQ ID No: 1, or a sequence having high identity with said sequence (e.g., 80% or more, 90% or more, 91% or more, 92% or more, 93% or more, 94% or more, 95% or more, 96% or more, 97% or more, 98% or more, or 99% or more).

Examples of reporter genes used in the present invention include fluorescent protein genes, luciferase genes, and chromogenic enzyme genes, but are not limited to these. Examples of luciferases coded by the reporter gene include firefly luciferase, bacterial luciferase, synthetic Renilla luciferase, Oplophorus gracilirostris luciferase (e.g., NanoLuc (registered trademark)), and secreted luciferases. Examples of fluorescent proteins coded by the reporter gene include green fluorescent proteins such as GFP and EGFP, blue fluorescent proteins such as BFP and TagBFP, and red fluorescent proteins such as RFP and DsRed. Examples of chromogenic enzyme genes coded by the reporter gene include β-galactosidase, β-glucuronidase, and alkaline phosphatase. These marker genes may be used alone or in combination.

The nucleic acid of the present invention preferably has a sequence that terminates transcription of mRNA from the reporter gene (also called polyadenylation (poly A) signal or terminator) downstream of the reporter gene. For example, a terminator sequence derived from a viral gene or various mammalian or avian genes can be used. Preferably, an SV40-derived terminator is used.

The nucleic acid of the present invention may further contain a drug resistance gene or another reporter gene, and cells into which said nucleic acid has been introduced can be selected based on drug resistance or reporter activity as an index. Examples of drug resistance genes include neomycin phosphotransferase II (nptII) gene, hygromycin phosphotransferase (hpt) gene, and puromycin-n-acetyltransferase (Pac) gene. Examples of other reporter genes include β-galactosidase (lacZ) gene and chloramphenicol acetyltransferase (cat) gene.

The above drug resistance or reporter gene is preferably under the control of any promoter that can function in cells. For example, viral promoters such as SV40-derived early promoter, cytomegalovirus (CMV) long terminal repeat (LTR), rous sarcoma virus (RSV) LTR, moloney murine leukemia virus (MoMuLV) LTR, and adenovirus (AdV)-derived early promoter, as well as β-actin gene promoter, PGK gene promoter, transferrin gene promoter, etc. can be used. Furthermore, the above drug resistance or other reporter gene also preferably has a poly A signal.

The reporter cell of the present invention can be produced by introducing the nucleic acid of the present invention into pluripotent stem cells, and inducing nail matrix cells from the pluripotent stem cells having the nucleic acid of the present invention. Alternatively, the reporter cell of the present invention can be produced by introducing the nucleic acid of the present invention into nail matrix cells or precursor cells thereof, and differentiating them as needed. It is preferable that the above nail matrix cells are obtainable by the production method of the present invention.

The method for introducing the nucleic acid of the present invention into cells is not particularly limited, but typically involves introduction into cells using an expression vector containing the nucleic acid of the present invention. The nucleic acid of the present invention may be incorporated into the genome of a cell or may exist independently of the genome. Examples of expression vectors that can be used in the present invention include viral vectors and plasmid vectors. Viral vectors include retroviral vectors (including lentiviral vectors and pseudotype vectors), adenoviral vectors, adeno-associated virus vectors, herpesvirus vectors, Sendai virus, and episomal vectors. A transposon expression system (e.g., PiggyBac system) may also be used. Examples of plasmid vectors include animal cell expression plasmids (e.g., pa1-11, pXT1, pRc/CMV, pRc/RSV, pcDNAI/Neo). In one embodiment, the nucleic acid of the present invention is introduced into cells using a PiggyBac system, which employs a transposon vector containing the nucleic acid of the present invention and a transposase expression vector expressing a transposase. The genes contained in the transposon vector and the transposase expression vector may be separated into separate vectors as described above, or they may be contained in a single vector. The transposon vector and the transposase expression vector can have, for example, the following configuration. In order to excise the nucleic acid of the present invention from the transposon vector by the transposase, the transposon vector contains terminal inverted repeat sequences (TIRs) upstream of the 5' end and downstream of the 3' end of said nucleic acid. The transposase recognizes the terminal inverted repeat sequences contained in the transposon vector and excises the nucleic acid of the present invention flanked by the terminal inverted repeat sequences from the transposon vector.

Alternatively, the reporter cell of the present invention can be prepared by knocking in a reporter gene downstream of the promoter of the endogenous Lgr6 gene on one or both chromosomes (alleles) using genome editing or homologous recombination. When utilizing homologous recombination, a donor DNA is typically used, and this donor DNA contains at least a reporter gene in the region between the 5' homology arm and the 3' homology arm (hereinafter also referred to as "region to be inserted"). The region to be inserted may be inserted downstream of the promoter of the Lgr6 gene on the chromosome, or it may replace the region downstream of said promoter (e.g., a region encoding Lgr6 or a part thereof). Furthermore, a fusion protein of a reporter protein and Lgr6 may be expressed, or an IRES (internal ribosome entry site) or 2A (e.g., T2A, P2A, E2A, F2A) coding sequence may be included in the region to be inserted to simultaneously express the reporter protein and Lgr6.

The genome editing includes, for example, a method using zinc finger nucleases (ZFNs), which are composed of a zinc finger DNA-binding domain linked to a non-specific DNA cleavage domain (JP Patent No. 4968498), a method using TALENs (TAL effector nucleases) composed of a transcription activator-like (TAL) effector, which is a DNA-binding module, linked to a DNA endonuclease (WO2011/072246), or a method utilizing a CRISPR-Cas9 system in which a DNA sequence CRISPR (Clustered Regularly interspaced short palindromic repeats) and a Cas protein family, which plays an important role together with CRISPR, are combined (WO2013/176772). Furthermore, the gene expression cassette may be knocked into a specific locus by genome editing or the like. As the specific locus, there is a locus such as the human PPP1R2C locus, which has an open chromatin structure and the expression of the inserted gene is less likely to be suppressed.

Any of the following methods can be used for introducing the nucleic acid of the present invention into cells: calcium phosphate co-precipitation, electroporation, lipofection, retroviral infection, aggregation method, microinjection, gene gun, and DEAE-dextran method.

### 5. Method for screening a prophylactic or therapeutic medicament for fingertip disorders

The reporter cell of the present invention may show a correlation between the protein expression level of Lgr6 and the expression level of the reporter protein. An increase in the protein expression level of Lgr6 reflects an increase in the protein expression of Lgr6 in nail matrix cells and/or proliferation of nail matrix cells. Furthermore, stimulation of the Wnt signaling pathway is important for the proliferation of nail matrix cells, and Lgr6 is known to stimulate the Wnt signaling pathway. Therefore, a substance that enhances the expression of Lgr6 can be a candidate substance for prophylactic or therapeutic medicaments for fingertip disorders. Therefore, the present invention also provides a method for screening a prophylactic or therapeutic medicament for fingertip disorders such as nail disorders using the reporter cell of the present invention (hereinafter referred to as "screening method of the present invention").

The screening method of the present invention comprises the following steps:
(1) contacting the reporter cell of the present invention with a test substance;
(2) measuring the expression level of the reporter protein in the cell; and
(3) selecting the test substance as a candidate for a prophylactic or therapeutic medicament for fingertip disorders when the expression level measured in step (2) is higher than that in a control group not contacted with the test substance, or higher than that measured in the absence of the test substance.

In the present invention, examples of the test substance include a cell extract, a cell culture supernatant, a microbial fermentation product, an extract derived from a marine organism, a plant extract, a purified or crude protein, a peptide, a non-peptide compound, a synthetic low-molecular-weight compound, a natural compound.

In the present invention, the test substance can also be obtained using any of many approaches in combinatorial library methods known in the art, including (1) a biological library, (2) a synthetic library method using deconvolution, (3) a "one-bead one compound" library method, and (4) a synthetic library method using affinity chromatography sorting. Examples of a method for synthesizing a molecular library can be found in the art (DeWitt et al. (1993) Proc. Natl. Acad. Sci. USA 90: 6909-13; Erb et al. (1994) Proc. Natl. Acad. Sci. USA 91: 11422-6; Zuckermann et al. (1994) J. Med. Chem. 37: 2678-85; Cho et al. (1993) Science 261: 1303-5; Carell et al. (1994) Angew. Chem. Int. Ed. Engl. 33: 2059; Carell et al. (1994) Angew. Chem. Int. Ed. Engl. 33: 2061; Gallop et al. (1994) J. Med. Chem. 37: 1233-51). The compound library can be produced as solutions (see Houghten (1992) Bio/Techniques 13: 412-21) or beads (Lam (1991) Nature 354: 82-4), chips (Fodor (1993) Nature 364: 555-6), bacteria (U.S. Patent No. 5,223,409), spores (U.S. Patent No. 5,571,698, U.S. Patent No. 5,403,484, and U.S. Patent No. 5,223,409), plasmids (Cull et al. (1992) Proc. Natl. Acad. Sci. USA 89: 1865-9), or phages (Scott and Smith (1990) Science 249: 386-90; Devlin (1990) Science 249: 404-6; Cwirla et al. (1990) Proc. Natl. Acad. Sci. USA 87: 6378-82; Felici (1991) J. Mol. Biol. 222: 301-10; U.S. Publication No. 2002103360).

The contact of the test substance with the reporter cell of the present invention in step (1) can be performed, for example, by adding the test substance to a medium or various buffers (e.g., HEPES buffer, phosphate buffer, phosphate-buffered saline, Tris-HCl buffer, borate buffer, acetate buffer, etc.) and incubating the cell for a certain period of time. The incubation time is, for example, 10 minutes to 24 hours, but is not limited to this range.

The measurement of the expression level of the reporter protein in the reporter cell of the present invention in step (2) can be performed by known methods. For example, if the reporter gene is a luciferase gene, it can be measured by luminescence intensity. If the reporter gene is a fluorescent protein gene, it can be measured by fluorescence intensity. If the reporter gene is a chromogenic enzyme gene, it can be measured by chromogenic intensity. In addition, expression of the reporter gene can be detected or measured using a fluorescence imaging system or a luminescence imaging system. More specifically, for example, when a luciferase gene is used as the reporter gene, the reporter cells and the test substance are cultured, the cell extract is obtained, the extract is reacted with luciferin and ATP to cause chemiluminescence, and the luminescence intensity is measured, thereby measuring the expression level of the luciferase gene. At this time, commercially available luciferase reaction detection kits such as PicaGene Dual Kit (registered trademark; manufactured by Toyo Ink) can be used. In addition, when a fluorescent protein gene is used as the reporter gene, the reporter cells and the test substance are cultured, the cells are washed, light of an excitation wavelength corresponding to the fluorescent protein used for the cells is irradiated onto the cells, and the intensity of light of the corresponding fluorescence wavelength is measured, thereby measuring the expression level of the fluorescent protein gene.

In step (3), "not contacted with the test substance" includes cases where a solvent (blank) having the same amount as the test substance is added instead of the test substance, or a negative control substance that does not affect the expression level of the reporter protein is added. In addition, criteria for selecting the test substance as a candidate for a prophylactic or therapeutic medicament for fingertip disorders can be appropriately determined by those skilled in the art. For example, in the above-described screening method, if the expression level of the reporter protein in the presence of the test substance is increased compared to the case where the test substance is not present (for example, increased by about 20% or more, preferably about 30% or more, and more preferably about 50% or more), the test substance can be selected as a candidate for a prophylactic or therapeutic medicament for fingertip disorders.

The present invention will be described more specifically with reference to the following examples. It is to be noted, however, that the present invention is not limited to these examples by any means.

### [EXAMPLES]

### Materials and Methods

### 1. Human iPSCs (induced pluripotent stem cells)

The hiPSC lines (RIKEN-2F and 253G1) were obtained from the Laboratory for Pluripotent Cell Studies, RIKEN BioResourse Research Center, Ibaragi, Japan.

### 2. Maintenance and digit organoid differentiation of human iPSCs

The human iPSCs were differentiated into limb bud organoids by reference to the previously reported methods with some modifications (Mori S, et al., Nat Commun. 2019;10: 3802). In this report, differentiation experiments for data acquisition were performed using the 253G1 cell line; however, RIKEN2F cells also differentiated into similar digit organoids (data not shown). Undifferentiated human iPSCs were maintained on plastic dishes (Corning Inc., NY, USA) coated with 0.5 g/cm² iMatrix511 Silk (Nippi Inc., Tokyo, Japan) using StemFit AK02N (Ajinomoto, Tokyo, Japan) as the culture medium. The dispersed cells were seeded at a density of 15,000 cells/cm² with a 10 µM Rho kinase inhibitor, Y-27632 (Sellec Inc., Tokyo, Japan). One day later, the medium was replaced with the same medium without Y-27632. Passages of the cells were performed every three to four days. The cells were detached from the culture dish by the treatment with TripLE express enzyme (Thermo Fisher Scientific, Waltham, MA, USA) supplemented with 10 µM Y-27632 for 20 min at 37°C. For differentiation, five days before the initiation of the differentiation (Day 0), the dispersed cells were dispersed into StemFit AK02N with a 10 µM Y-27632 and distributed to a non-cell adhesive 96 well plate (Sumitomo Bakelite, Tokyo, Japan) at a density of 3500 cells per well with 200 µL of the medium. One day before Day 0, aspirate 100µL of medium from all wells and add 100 µL Essential 8 (E8) medium (Thermo Fisher Scientific) with a 10 µM Y-27632. On Day 0, half of medium was exchanged with Essential 6 (E6) medium (Thermo Fisher Scientific) containing 20 ng/ml BMP4, and added to 96 wells. On Day 2, 3, and 6 of differentiation, each 100 µl of the medium was aspirated from all wells, and each 100 µL of E6 medium with 10 ng/ml BMP4 was supplied. On Day 8 of differentiation, each 100 µL of the medium was aspirated from all wells, and each 100 µL of Dulbecco's modified Eagle medium (DMEM) (Fujifilm Wako Pure Chemical Corporation) supplemented with a 1/100 dilution of the mix of insulin, transferrin, and selenium (ITS, Thermo Fisher Scientific), and 10 ng/ml BMP4 was added. On Day 9, half of the culture medium was replaced with DMEM supplemented with a 1/100 ITS, 10 ng/ml BMP4, and 2 µM all-trans retinoic acid (RA). On Day 10, half of the culture medium was replaced with DMEM supplemented with 1/100 ITS, 10 ng/ml BMP4, and 2 µM LDN-193189. On Day 11, half of the culture medium was replaced with DMEM supplemented with 1/100 ITS and 10 ng/ml BMP4. On Day 13, all spheres were collected from a 96-well plate using a 1000 µL wide-bore chip (Axygen Corning Inc.) and transferred to a cell-non adhesive 10-cm dish (Cell-Repellent Surface, Greiner Bio-One Kremsmünster, Austria) with 20 ml of organoid maturation medium (OMM) consisting of a 1:1 mixture of DMEM/F12 (Fujifilm Wako Pure Chemical Corporation, Miyazaki, Japan) and Neurobasal medium (Thermo Fisher Scientific), supplemented with 0.1 mM non-essential amino acids, 1 mM glutamine, 55 µM 2-mercaptoethanol, 0.5% N-2 supplement (Thermo Fisher Scientific), 0.5% B-27 supplement (Thermo Fisher Scientific), 50 µg/mL ascorbic acid 2-phosphate (Merck KGaA, Darmstadt, Germany), 0.05% bovine serum albumin (Merck) supplemented with 7 µM CHIR-98014 (Sellec Inc.), 10 ng/mL FGF-basic (Nacalai Tesque Inc., Kyoto, Japan), 10 ng/ml FGF8 (Peprotech, Thermo Fisher Scientific), and 4 µg/ml hydrocortisone (Tokyo Chemical Industry Co., Ltd., Tokyo, Japan), and started gyratory rotation culture with approximately 60 rpm of agitation-speed until the end of the culture experiment with changing medium every third day.

### 3. Lgr6-GFP reporter iPS development

We chose the tentative promoter region as 1 kb of upstream sequence from the first codon of *Lgr6 variant 2* (GenBank: AL356953.17 from 19771 to 20838). The Kozak sequence (GCCACC) was inserted just above the upstream of the first codon of EGFP. The piggyBac-based reporter vector has 5' and 3' inverted terminal repeat sequences (ITRs), and between them, has *Lgr6* promoter-Kozak-*EGFP*-rabbit beta globin (rBG) polyA signal, and cytomegalovirus promoter (CMV)-*puromycin resistant gene*-bovine growth factor (BGH) polyA signal sequences. The designed vector was constructed and purchased from VectorBuilder (VectorBuilder Inc., Kanagawa, Japan). We transduced the construct into 253G1 and RIKEN2F cell lines with a piggyBac transposase expression vector (System Biosciences, LLC., CA, USA). Three to four days later, the gene-transduced cells were selected by two to three days' treatment with 2 µg/ml puromycin (Thermo Fisher Scientific) and subsequently cultured to form single cell-derived colonies for several days. We manually picked individual colonies and further expanded them for functional tests. We performed small-scale digit organoid differentiation and successfully selected functional clones through fluorescent microscopic observations.

### 4. Quantitative polymerase chain reaction (qPCR) analysis

Total RNA was extracted from the cells using ISOGEN (NIPPON GENE CO., LTD., Tokyo, Japan) according to the manufacturer's instructions. Reverse transcription of 50 ng of RNA was performed using SuperScript Reverse Transcriptase (Thermo Fisher Scientific) with an oligo(dT)20 primer. To investigate various gene expressions, a quantitative polymerase chain reaction was performed using GeneAce SYBR qPCR Mix α (NIPPON GENE CO., LTD.) with gene-specific primer sets listed in Table 1. The expression levels of the other genes were also investigated. All gene expression levels were normalized to internal ribosomal protein S18 expression levels. The sequences in Table 1 are shown in the sequence listing, with SEQ ID Nos: 2 to 19, in ascending order from top to bottom.

**[Table 1]**

| Primers for qPCR | | |
|---|---|---|
| **gene name** | | **Sequence (from 5' to 3')** |
| *Hoxd13* | Fw | AGGTGTACTGCACCAAGGACCA |
| | Rv | GCAGTTTGGTGTAAGGCACTCTC |
| *Tbx5* | Fw | AAATGAAACCCAGCATAGGAGCTGGC |
| | Rv | ACACTCAGCCTCACATCTTACCCT |
| *Pitx1* | Fw | GTACGCACTTCACAAGCCAGCA |
| | Rv | GCTCGGTGAGGTTGGTCCACA |
| *Runx2* | Fw | CCCAGTATGAGAGTAGGTGTCC |
| | Rv | GGGTAAGACTGGTCATAGGACC |
| *Krt-17* | Fw | GGTGGGTGGTGAGATCAATGT |
| | Rv | CGCGGTTCAGTTCCTCTGTC |
| *Krt-81* | Fw | GCATTGGGGCTGTGAATGTCT |
| | Rv | ACCCAGGGAGCTGATACCAC |
| *Lgr5* | Fw | GTTTCCCGCAAGACGTAACT |
| | Rv | CAGCGTCTTCACCTCCTACC |
| *Lgr6* | Fw | TGACGGCTTACCTGGACCTCA |
| | Rv | AGAGAATGCTTGTCCTGGGATG |
| *Ribosomal protein 18s* | Fw | GCGGCGGAAAATAGCCTTTG |
| | Rv | GATCACACGTTCCACCTCATC |

### 5. Immunofluorescent staining

Organoids were fixed in 4% paraformaldehyde for 20 minutes at 25 °C. The organoids were then washed twice with Tris-buffered saline containing 0.2% Tween-20 (TBS-T) and incubated with 0.1% Triton-X100 containing TBS-T for 10 minutes at 25 °C, followed by immersion in 30% sucrose containing TBS-T for overnight or longer at 4 °C. Organoids were settled on the bottom of the cup (Cryomold No. 1, Sakura Finetek Japan Co., Ltd., Tokyo, Japan), and then the solution was aspirated completely by the paper absorption (Kimwipe, Nippon Paper Crecia Co., Ltd., Tokyo, Japan) from the pinhole made by the tip of a 29G needle. Subsequently, the cup was filled with O.C.T. compound (Sakura Finetek Japan Co., Ltd.) and cooled to -80 °C for making cryoblocks. The cryoblocks were applied to 8 µm thick sectioning and adhered onto aminosilane treated slide glass (Matsunami Glass IND., Ltd., Osaka, Japan). After being well dried, the slide glasses were dipped in TBS-T to wash away the residual O.C.T. compound and rehydrate the organoid. Then, the slides were treated with a blocking solution (Nacalai Tesque) for 30 minutes at 25 °C. Primary antibody-containing blocking solution was applied and incubated overnight at 4 °C with paraffin sealing to prevent evaporation. The slide glasses were then washed three times with TBS-T and immersed in a blocking solution containing the secondary antibody for 1 hour at 25 °C. After washing thrice, fluorescent signals were observed using a fluorescence microscope (Eclipse Ti2, Nikon Instruments, Tokyo, Japan) controlled by the equipped software (NIS-Elements, Nikon Instruments). The primary and secondary antibodies used are listed in Table 2. For the anti-Lgr5 antibody, rabbit antibody (GENETEX GNT-GTX130204-25) was used at a dilution of 1:200. In Example 4, the anti-Lgr6 antibody was also used at a dilution of 1:200.

**[Table 2]**

| The antibodies used in this study | | | | | |
|---|---|---|---|---|---|
| Antigen Name | Host | Clonality | Manufacturer | I.D. | Dilution |
| Brachyury | Goat | polyclonal | Santacruz | SC-17743 | 1:100 |
| E-cadherin | Rabbit | polyclonal | Santacruz | SC-7870 | 1:100 |
| Hand2 | Mouse | monoclonal | Santacruz | SC-398167 | 1:100 |
| Pitx1 | Mouse | monoclonal | Santacruz | SC-271435 | 1:100 |
| Lgr6 | Mouse | monoclonal | Santacruz | SC-393010 | 1:100 |
| Keratin-17 | Mouse | monoclonal | Santacruz | SC-393002 | 1:100 |
| Keratin-81 | Mouse | monoclonal | Santacruz | SC-100929 | 1:100 |
| GFP | Goat | polyclonal | Santacruz | SC-5385 | 1:100 |
| | | | | | |

| Product name | Host | Clonality | Manufacturer | I.D. | Dilution |
|---|---|---|---|---|---|
| rabbit IgG(H+L) AlexaFluo488 | Donkey | polyclonal | Thermo Scientific | A21206 | 1:200 |
| mouse IgG(H+L) AlexaFluo546 | Donkey | polyclonal | Thermo Scientific | A10036 | 1:200 |
| goat IgG(H+L) AlexaFluo546 | Donkey | polyclonal | Thermo Scientific | A11056 | 1:200 |
| goat IgG(H+L) AlexaFluo488 | Donkey | polyclonal | Thermo Scientific | A11055 | 1:200 |

### 6. Statistical analysis

Statistical analyses were performed using EZR software (Jichi Medical University, Japan). For comparisons with differentiation day 0, significant differences were determined using one-way analysis of variance (one-way ANOVA), followed by post-hoc testing using the Dunnett test. Statistical significance was set at P < 0.05.

### Example 1: Differentiation of digit organoids via the extended culture of limb bud organoids with gyratory rotation

To differentiate digit organoids from human induced pluripotent stem cells (iPSCs), a previous report describing limb bud differentiation from human iPSCs was referenced and the method was modified and extended (NPL 1). iPSC spheres were sequentially subjected to suspension culture of limb bud organoids. A gyratory culture was adopted for further growth and differentiation of the organoids (Fig. 1A). E-cadherin, brachyury, Hand2, and Pitx1 were sequentially expressed, confirming the successful early differentiation of the limb bud organoids (Fig. 1B). These results confirmed that limb bud differentiation was successfully reconstituted.

### Example 2: mRNA expression of segment patterning and digit-related genes during gyratory rotation culture

In an attempt to perform long-term culture of the organoids, it was found that organoids that had grown larger possessed necrotic cores in static suspension culture. Therefore, a continuous flow of medium was maintained during the extended culture period. Gyratory culture was performed for further growth and differentiation of the organoids. The expression levels of mRNAs of various key genes related to arm segmentation, skeletal bone formation, nail matrix differentiation, and nail formation were investigated (Fig. 2). Hoxd13, a transcription factor involved in digit formation, peaked in expression on differentiation Day 49, indicating successful differentiation of the most proximal digit segment. Pitx1 was continuously expressed in differentiated mesenchymal cells. Runx2 expression was elevated on differentiation Day 49 and markedly increased on Day 77. Runx2 protein is involved in skeletal bone formation and supports further progression of the differentiation process into the bone formation phase. Lgr5-expressing cells were reported to be present in the hair follicle and on the dorsal side of the proximal nail fold (Lehoczky JA, Tabin CJ. Proc Natl Acad Sci U S A. 2015;112: 13249-13254.). Lgr5 expression was sharply increased on differentiation Day 14. However, it rapidly decreased thereafter and gradually increased again with increasing differentiation time. In contrast, Lgr6 expression gradually and continuously increased with differentiation. The expression of typical nail keratins, KRT17 (type I; also known as CK-17 and PCHC1) and KRT81 (type II; also known as hHb1, MLN137, ghHkb1, and hHAKB2-1), gradually increased with differentiation.

Type I keratin KRT17 and type II keratin KRT81 are known as hard keratins because they form hair and nails by creating heterocomplexes. Additionally, Lgr6-positive cells or Lgr5-positive cells are known to also be stem cell populations of hair follicles (Jaks V. et al., Nat Genet. 2008;40: 1291-1299.; Snippert HJ. et al., Science. 2010;327: 1385-1389.). Therefore, it may be considered difficult to determine whether the cells contained in the obtained organoids are hair follicle stem cells or nail matrix cells based solely on the aforementioned molecular markers. However, for the following reasons, it can be determined that the cells obtained by the production method of the present invention are nail matrix cells. First, the developmental process until nail formation was traced, and a time-dependent increase in the digit-specific Hoxd13 was observed (Fig. 2). On the other hand, fetuses normally do not have active hair follicles in their arms or digits (Domagala Z. et al., Adv Clin Exp Med. 2017;26: 967-972.). Furthermore, immunohistochemical analysis indicated the absence of hair follicle features over a relatively wide area. These results strongly support the inventor's hypothesis that the differentiated tissue in the digit organoids of the present invention is a combination of nail matrix and nail. The second reason is the major difference from a previous study on hair and hair follicles using organoids derived from human embryonic stem cells (Lee J. et al., Nature. 2020;582: 399-404.). Lee et al. reported hair-bearing human skin produced by long-term culture of skin organoids and claimed that the skin organoids were equivalent to human facial skin. Notably, their single-cell mRNA expression analysis revealed that the organoids consisted only of ectodermal cells, with no endodermal or mesodermal populations. In contrast, the digit organoids of the present invention clearly contained mesodermal cells. The initial spheres were richly populated with brachyury-positive mesodermal mesenchymal cells (Fig. 1B). In the later phase of differentiation, cells expressing Runx2 mRNA, which might be osteoblasts forming skeletal bone (Karsenty G., Endocrinology. 2001;142: 2731-2733.), appeared (Fig. 2). From the above, it was concluded that the differentiated digit organoids of the present invention contain nail matrix cells and primitive nail tissue.

### Example 3: Neighboring protein expressions of Lgr6, KRT17, and KRT81 in digit organoids

Gyratory rotation culture was maintained during the period when the human embryo develops primitive digits. Serial sections of one organoid were used to confirm the expression of Lgr6, KRT17, and KRT81 proteins at adjacent sites (Fig. 3).

### Example 4: Verification of the relationship between Lgr5-positive cells and Lgr6-positive cells

Organoids at day 62 after the initiation of differentiation were used to detect Lgr5-positive cells and Lgr6-positive cells. As a result, it was shown that Lgr5 protein-expressing cells were present in the vicinity of Lgr6 protein-expressing cells, and cells co-expressing both were also present (Fig. 4). Therefore, a strong relationship between Lgr5-positive cells and Lgr6-positive cells is suggested.

### Example 5: Development of a functional human Lgr6-GFP reporter cell line

The Eukaryotic Promoter Database (EPD) was searched, and three *Lgr6* alternative splicing variants were identified. Because the clearest signal was observed in variant 2 by the Cap Analysis of Gene Expression (CAGE) analysis of FANTOM5 and ENCODE, about 1 kb of upstream sequence from the first codon of variant 2 was selected for the *Lgr6* reporter construct (Fig. 5A). Several clones of gene-transduced human iPSCs were established, and digit organoid formation was demonstrated in parallel for screening. Some of the clones showed green fluorescence signals (Fig. 5B). Immunofluorescent staining for GFP and Lgr6 was performed on cryosections of digit organoids. Co-localization of reporter GFP and Lgr6 proteins was confirmed (Fig. 5C).

### Example 6: Comprehensive gene expression analysis of digit organoids

RNA sequencing was performed on cells at Day 0 (undifferentiated iPS cells) and digit organoids at Day 50 (samples not used for qPCR) to comprehensively compare gene expression. RNA (total RNA including ribosomal RNA) was extracted and quantified from undifferentiated iPS cells (RIKEN2F strain) and samples of digit organoids derived from the same strain at Day 50 of differentiation. 109 ng of total RNA sample was processed with the NEBNext Poly(A) mRNA Magnetic Isolation Module (NEB) to enrich poly-A mRNA and remove rRNA molecules. cDNA synthesis and subsequent transcriptome library preparation were performed using the NEBNext Ultra II Directional RNA Library Prep Kit for Illumina (NEB). To increase the library amount and incorporate sample barcodes into the library fragments, 13 cycles of PCR amplification were performed. The resulting transcriptome library was loaded onto the NovaSeq 6000 next-generation sequencing platform (Illumina). Sequencing was performed in a 150 bp paired-end (PE) configuration according to the manufacturer's instructions, and approximately 6 GB per sample was obtained. Low-quality raw reads and adapter sequences were removed using Cutadapt v.2.9. The remaining reads were mapped to the human genome (GRCh38 version 101) using HISAT2 v.2.2.0 (PMID: 25751142), and gene expression was quantified using featureCounts v.2.6.0 (PMID: 24227677). To eliminate effects derived from transcript length, reads per 1,000 bp of transcript were calculated and normalized. Normalization by the total number of reads was performed to obtain FPKM (fragments per kilobase of exon per million reads mapped). In addition, after removing genes with zero read counts using DESeq2 v.1.6.3, differentially expressed genes (DEGs) were identified based on differences in expression levels between samples (log2 fold change > 1 and adjusted p value < 0.05).

From the DEG list, genes with logFC > 10 or logFC < -10 were selected, and further narrowed down to a false discovery rate (FDR) ≤ 0.1, resulting in 329 genes (226 up-regulated genes and 103 down-regulated genes) that changed significantly (data not shown). A volcano plot was created for genes outside the logFC threshold of ±10 (Fig. 6). From the DEG list, genes related to digit organoids were listed by category, and graphs were created (Figs. 7 and 8). From these data, it became clear that in the digit organoids, both Lgr5 and Lgr6 genes, homeobox genes such as HoxD12 and HoxD13, and other gene groups important for arm development were all up-regulated. In addition, many keratin genes were also up-regulated. Thus, these data are consistent with the results of the real-time PCR in Example 2. Furthermore, in the digit organoids, transcription factors associated with the undifferentiated state were almost no longer expressed.

### Example 7: Transplantation of digit organoids into mice

Digit organoids were prepared by a method similar to that of Example 5, and cells in which green fluorescence of GFP was observed with an Lgr6 reporter (GFP-positive cells) were collected using a cell sorter (FACS Aria IIIBD) (Fig. 9A). In cell-nonadherent 96-well round-bottom flasks (SUMILON MS-9096U, M), cell aggregates of 100 to 1000 cells each were formed (Fig. 9A). The cell aggregates were transplanted subcutaneously near the site where nail matrix cells are present or into the subcutaneous tissue of the back in NOD-SCID mice (immunodeficient mice). Anesthesia was performed by exposing the mice to 5% vaporized Forane. Digit organoids prepared by a method similar to that of Example 5 may also be transplanted as intact cell aggregates into the subcutaneous tissue near the site where nail matrix cells are present or into the subcutaneous tissue of the back of NOD-SCID mice. Furthermore, the digit organoids may be embedded in Matrigel (BD), AteloCell (registered trademark) (KOKEN), or the like, and then transplanted.

Sections of cell aggregates formed 20 days after transplantation of the Lgr6-positive cell aggregates prepared by the above method into dorsal subcutaneous tissue are shown in Figs. 9B and 9C. Fig. 9B shows that host mouse erythrocytes were present surrounded by transplanted human cells, indicating successful engraftment via host circulation. Fig. 9C shows that Lgr6 reporter GFP was present in the transplanted human cells (hNA-positive cells), and that hard keratins KRT17 and KRT81, which are components of the nail, were co-localized in the vicinity.

Furthermore, a histological image 20 days after transplantation of approximately 300 Lgr6-positive cell aggregates prepared by the above method into the fingertips of immunodeficient mice is shown in Fig. 10. Fig. 10 shows that cells immunostained with an antibody against GFP in the Lgr6 reporter were present, and that they survived in the fingertip tissue and nail matrix region.

### [INDUSTRIAL APPLICABILITY]

According to the present invention, it becomes possible to provide nail matrix cells or digit organoids *in vitro*, and it becomes possible to develop regenerative medicine by transplanting said nail matrix cells or digit organoids into patients having fingertip disorders such as nail disorders. In particular, nail regeneration can be extremely important for the QOL of patients having nail disorders. Furthermore, according to the present invention, nail matrix cells having a nucleic acid containing a reporter gene under the control of a transcriptional regulatory region of the Lgr6 gene can be provided, and screening for preventive or therapeutic agents for fingertip disorders using said nail matrix cells also becomes possible. Therefore, the nail matrix cells of the present invention are extremely useful in the medical field, and particularly in the field of regenerative medicine.

The present application claims priority based on Japanese Patent Application No. 2023-214532 filed in Japan (filing date: December 20, 2023), the disclosure of which is incorporated herein in its entirety by reference.

## Claims

1. A method for producing a nail matrix cell, comprising a step of culturing a nail stem cell by shaking under suspension culture conditions in the presence of a Wnt signaling activator, an FGF signaling activator, and an adrenocortical hormone.

2. The method according to claim 1, wherein the nail stem cell is contained in a limb bud organoid.

3. The method according to claim 1 or 2, wherein the nail matrix cell is contained in a digit organoid.

4. The method according to any one of claims 1 to 3, wherein the Wnt signaling activator is CHIR98014.

5. The method according to any one of claims 1 to 4, wherein the FGF signaling activator is bFGF and/or FGF8.

6. The method according to any one of claims 1 to 5, wherein the adrenocortical hormone is hydrocortisone.

7. The method according to any one of claims 1 to 6, wherein the nail stem cell is derived from a pluripotent stem cell.

8. The method according to claim 7, wherein the pluripotent stem cell is derived from a human.

9. A nail matrix cell or a digit organoid, comprising a cell producible by the method according to any one of claims 1 to 8.

10. An agent for treating fingertip disorders, comprising the nail matrix cell or the digit organoid according to claim 9.

11. The agent according to claim 10, wherein the fingertip disorders are nail disorders.

12. A nail matrix cell having a nucleic acid comprising a reporter gene under the control of a transcriptional regulatory region of an Lgr6 gene.

13. The nail matrix cell according to claim 12, producible by the method according to any one of claims 1 to 8.

14. The nail matrix cell according to claim 12 or 13, wherein the reporter gene is a gene encoding a fluorescent protein.

15. A method for screening a prophylactic or therapeutic medicament for fingertip disorders, comprising the following steps (1) to (3):
(1) contacting the nail matrix cell according to any one of claims 12 to 14 with a test substance,
(2) measuring the expression level of the reporter protein in the cell, and
(3) selecting the test substance as a candidate for a prophylactic or therapeutic medicament for fingertip disorders when the expression level measured in step (2) is higher than that in a control group not contacted with the test substance, or higher than that measured in the absence of the test substance.
